# EUROPEAN PATENT SPECIFICATION

(11) **EP 2 652 501 B1**
(45) Date of publication and mention of the grant of the patent: **20.04.2016**
(21) Application number: 11793466.1
(22) Date of filing: 12.12.2011
(51) Int. Cl.: G01N 33/564

(54) **METHOD FOR DIAGNOSIS, MONITORING THE EFFICACY OF A THERAPY AND FOR DEVELOPMENT OF TREATMENT FOR MULTIPLE SCLEROSIS**
VERFAHREN ZUR DIAGNOSE, ÜBERWACHUNG DER WIRKSAMKEIT EINER THERAPIE UND ZUR ENTWICKLUNG EINER BEHANDLUNG FÜR MULTIPLE SKLEROSE
MÉTHODE DE DIAGNOSTIC DE LA SCLÉROSE EN PLAQUES, DE CONTRÔLE DE L'EFFICACITÉ D'UN TRAITEMENT ET DE DÉVELOPPEMENT D'UN TRAITEMENT DE LA SCLÉROSE EN PLAQUES

(30) Priority: 13.12.2010 IT RM20100654
(43) Date of publication of application: 23.10.2013
(73) Proprietor: Avvedimento, Vittorio Enrico, 81100 Caserta (IT); Paterno', Roberto, 80132 Napoli (IT); Santillo, Mariarosaria, 80129 Napoli (IT)
(72) Inventor: Avvedimento, Vittorio Enrico, 81100 Caserta (IT); Paterno', Roberto, 80132 Napoli (IT); Santillo, Mariarosaria, 80129 Napoli (IT)
(74) Representative: Capasso, Olga
(86) International application number: PCT/EP2011/072491
(87) International publication number: WO 2012/080193

(56) References cited:
- WO-A1-2008/090213
- HAIYAN HU ET AL: "Role of CREB in the regulatory action of sarsasapogenin on muscarinic M1 receptor density during cell aging", FEBS LETTERS, vol. 584, no. 8, 1 April 2010 (2010-04-01) , pages 1549-1552, XP055125006, ISSN: 0014-5793, DOI: 10.1016/j.febslet.2010.03.006

## Description

### Field of the invention

The present invention relates to a method to diagnose, and/or monitor the efficacy of a therapy and/or screen for a therapeutic agent for multiple sclerosis based on the measurement of expression or localization of specific proteins, involved in human oligodendrocytes differentiation.

### Background to the invention

Multiple sclerosis (MS) is characterized by inflammation, demyelination and gliosis that damage the central nervous system (CNS) (Noseworthyet al., 2000; Trapp et al., 1998). MS is the most common cause of disability in young adults and is one of the most debilitating medical conditions, not only physically, but also on psycho-social levels. As in other chronic inflammatory diseases, the manifestations of MS change from a benign to a rapidly progressive and debilitating form. Some indirect data suggest an autoimmune etiology for multiple sclerosis, perhaps triggered by a viral infection, in a genetically susceptible individual (Marrie, 2004). Despite the number of studies on the disease and a multidisciplinary approach to the problem, the exact pathogenic mechanisms of multiple sclerosis are still obscure and the aetiology is unknown.

MS has no definitive diagnostic tests rendering necessary to use different diagnostic tools: clinical (Trojano and Paolicelli, 2001), laboratory (Luqueand Jaffe, 2007) and instrumental investigations (Young et al., 1981; Achtenand Deblaere, 2008).

The MS therapies currently available (beta-interferon, steroids, symptomatic therapies) act on the symptomatology and are aimed to slow the progression of the disease (Murdoch and Lyseng-Williamson, 2005). Thus, the early diagnosis of MS is needed because an earliest possible therapeutic intervention would be most effective in the long term.

Previous studies have shown that in MS patients chronic demyelinating lesions, there is a small number of pro-oligodendrocyte (pro-OL) and an increased number of oligodendrocyte progenitor cells (OPCs). This suggests that in pathological conditions, the OPCs maturation process is slower (Chang et al., 2002; Wolswijk, 1998).

This data is actually based on immunocytochemistry and immunohistochemistry investigations of the brain tissue. Therefore, there is the need to develop tools that evaluate the differentiation mechanisms at a molecular level and not at the organ level.

Previous studies showed that oligodendrocytes and their precursors are very sensitive to oxidative stress and this sensitivity is inversely correlated to the maturation level (Fragosoet al., 2004). The authors evaluated the effects of chronic stimulation of MO3-13 cells with low doses of hydrogen peroxide on the expression of oligodendrocyte differentiation markers.

The patent application WO 2008/090213 discloses that P-ERK1/2 or α-SMA may be used in the diagnosis of multiple sclerosis.

### Brief Description of the invention

The present invention relates to a method to diagnose and/or monitor the efficacy of a therapy and/or for the and/or screen for a therapeutic agent for multiple sclerosis based on the measurement of expression or localization of specific proteins, involved in human oligodendrocytes differentiation.

Changes of expression or localization of specific proteins involved in oligodendrocyte differentiation were measured after incubation of differentiating cells with cerebrospinal fluid, immunoglobulins extracted from blood serum or blood of patients with multiple sclerosis.

On this basis, it is possible, by using acellular system: i) to diagnose multiple sclerosis; ii) to screen drugs, for treatment of multiple sclerosis, already used in clinical therapy or in preclinical development; iii) to follow up treatment with drugs already used to treat multiple sclerosis.

Human oligodendrocyte MO3-13 cells differentiate when grown in the absence of serum and in the presence of PhorbolMyristate Acetate (PMA), also known as 12-O-Tetradecanoylphorbol-13- acetate (TPA). Expression or localization of specific differentiation markers was evaluated at different periods by western blotting, flow cytometry, real time PCR and immunofluorescence confocal microscopy.

The authors found that differentiation of these cells is accompanied by significant changes in specific intracellular markers. In addition, they have set up a new protocol to differentiate human oligodendrocytes. The cells are incubated for 30min-4 days in complete medium in the presence of low doses (200 µM) of hydrogen peroxide. Then, expression or localization of specific differentiation markers is evaluated. H₂O₂ produces the same changes of differentiation markers as PMA treatment.

In human oligodendrocyte incubated with CSF from multiple sclerosis (MS) patients, the authors found an inhibition of the differentiation induced by PMA, as indicated by differential expressions and/or cell localization of specific intracellular differentiation markers.

The authors obtained the same results, i.e. inhibition of differentiation, incubating the human oligodendrocyte with IgGs from blood serum from MS patients.

The authors found that in mouse cortical primary neurons and in HEK293 cells, the incubation with CSF and IgG from blood serum from MS patients does not produce any significant effect on the intracellular differentiation markers. This demonstrates that the effects of biological fluids from MS patients specifically target oligodendrocyte cells.

The results obtained testing 63 neurologic and 48 multiple sclerosis patients show that this assay, performed by measuring the intracellular differentiation markers phospho-Cyclic AMP Response Element Binding Protein (P-CREB), Extracellular Signal-Regulated Kinases 1/2 (P-ERK1/2), α-Smooth Muscle Actin (α-SMA), Myelin Basic Protein (MBP), Oligodendrocyte Transcription Factor 2 (olig-2) and muscarinic Acetylcholine Receptor M1 (mAchR M1) has a good sensitivity and specificity and is very reproducible.

The present data provide evidence for the development of a test to detect, at an early stage, the changes in intracellular signalling involved in oligodendrocytes differentiation, opening a new scenario for the development of an early and specific functional diagnosis for multiple sclerosis.

It is therefore an object of the invention a method for diagnosis and/or prognosis of multiple sclerosis in a subject,comprising the steps of:
a) incubating oligodendrocyte cells in the presence of a differentiation stimulus with a suitable amount of a biological sample obtained from the subject;
b) measuring the amount of one or more molecular species in said incubated oligodendrocyte cells, one of said molecular species being mAChR M1;
c) comparing the measured amount of said molecular species to a proper control and optionally
d) assessing the cellular localization of olig-2 and comparing the cellular localization of olig-2 to a proper control.

The present invention also provides a method for monitoring the efficacy of a therapeutic agent and/or screening for a candidate therapeutic agent for multiple sclerosis, comprising the steps of:
a) incubating oligodendrocyte cells in the presence of a differentiation stimulus with a suitable amount of a biological sample obtained from the subject, and with a therapeutic agent or a candidate therapeutic agent, respectively, for multiple sclerosis;
b) measuring the amount of one or more molecular species in said incubated oligodendrocyte cells, one of said molecular species being mAChR M1;
c) comparing the measured amount of said molecular species to a proper control and optionally
d) assessing the cellular localization of olig-2 and comparing the cellular localization of olig-2 to a proper control.

Preferably the methods further comprise measuring the amount of one or more molecular species belonging to the group of: olig-2, P-CREB, MBP or an introduced detectable marker gene under the control of one promoter selected from the group of mAChR M1, olig-2, P-CREB, MBP, genes and comparing the measured amount of said molecular species to a proper control.

Still preferably, the amount of at least two molecular species is measured.

In a preferred embodiment the amount of at least three molecular species is measured.

In a still preferred embodiment the amount of all of molecular species is measured.

In a yet preferred embodiment the detectable marker is a luciferase or GFP.

Preferably the method of the invention further comprises measuring the amount of at least another molecular species belonging to the group of: P-ERK1/2 or α-SMA.

Preferably the biological sample is cerebrospinal fluid, blood sample or serum sample or Ig-comprising derivatives thereof.

Still preferably the differentiation stimulus consists of incubating oligodendrocyte cells in the presence of: a) Phorbol Myristate Acetate (PMA); b) hydrogen peroxide; c) low serum medium; d) cyclic adenosine monophosphate (cAMP) analogs; e) adenylate cyclase activators; f) thyroid hormones as 3,5,3'-L-triiodothyronine (T3) and thyroxin (T4); g) ERB B inhibitors; h) nuclear receptor ligand; or i) nucleoside analogs.

It is a further object of the invention the use of a kit for the diagnosis and/or prognosis of multiple sclerosis or to monitor the efficacy of a therapy for multiple sclerosis according to the method of the invention comprising means to measure mAChR M1 and P-CREB.

Preferably said kit further comprises means to measure olig-2 and/or MBP and/or P-ERK1/2 and/or α-SMA.

In the case of a method for diagnosis and/or prognosis of multiple sclerosis, the control amount or localization may be the amount or localization measured or assessed in oligodendrocyte cells incubated with a sample taken from a healthy patient or from a patient affected by another neurological disease.

In the case of a method for monitoring the efficacy of a therapeutic agent, the control amount or localization may be the amount or localization measured or assessed in oligodendrocyte cells incubated with a sample taken from the same subject before the start of the therapy or at various time point throughout the course of the therapeutic agent.

In the case of a method to screen for a candidate therapeutic agent for multiple sclerosis, the control amount or localization may be the amount or localization measured or assessed in oligodendrocyte cells incubated with a sample taken from a positive (multiple sclerosis) patient without candidate therapeutic agent or with a reference treatment. In the present invention, two markers may be measured in the same incubated oligodendrocyte cells (simultaneously or subsequently), said markers being selected from the group of: P-CREB, P-ERK1/2, α-SMA, MBP, muscarinic acethylcholine receptor (mAchR) M1 or olig-2 and/or assessing the cellular localization of olig-2. Any combination is suitable for the purpose of the invention, for instance measuring P-CREB and assessing the cellular localization of olig-2, or measuring P-ERK1/2 and α-SMA or measuring MBP and assessing the cellular localization of olig-2 or measuring P-ERK1/2 and P-CREB, or measuring MBP and P-CREB etc...

Three markers may be measured in the same incubated oligodendrocyte cells (simultaneously or subsequently), said markers being selected from the group of: P-CREB, P-ERK1/2, α-SMA, MBP, muscarinic acethylcholine receptor (mAchR) M1 or olig-2 and/or assessing the cellular localization of olig-2. Any combination is suitable, for instance measuring P-CREB and α-SMA and assessing the cellular localization of olig-2, or measuring P-ERK1/2 and MBP and α-SMA, etc...

Four markers may be measured in the same incubated oligodendrocyte cells (simultaneously or subsequently), said markers being selected from the group of: P-CREB, P-ERK1/2, α-SMA, MBP, muscarinic acethylcholine receptor (mAchR) M1 or olig-2 and/or assessing the cellular localization of olig-2. Any combination is suitable, for instance measuring P-CREB, MBP, α-SMA and assessing the cellular localization of olig-2, or measuring P-ERK1/2 and MBP and α-SMA and P-CREB, ect...

All markers may be measured in the same incubated oligodendrocyte cells (simultaneously or subsequently), said markers being selected from the group of: P-CREB, P-ERK1/2, α-SMA, MBP, muscarinic acethylcholine receptor (mAchR) M1 or olig-2 and/or assessing the cellular localization of olig-2.

A further object is an in vitro method to differentiate oligodendrocyte precursor cells in oligodendrocyte cells comprising exposing said oligodendrocyte precursor cells to an effective amount of hydrogen peroxide. Preferably the amount of hydrogen peroxide is 100-200µM.

The invention will be now illustrated by means of examples referring to the following figures.

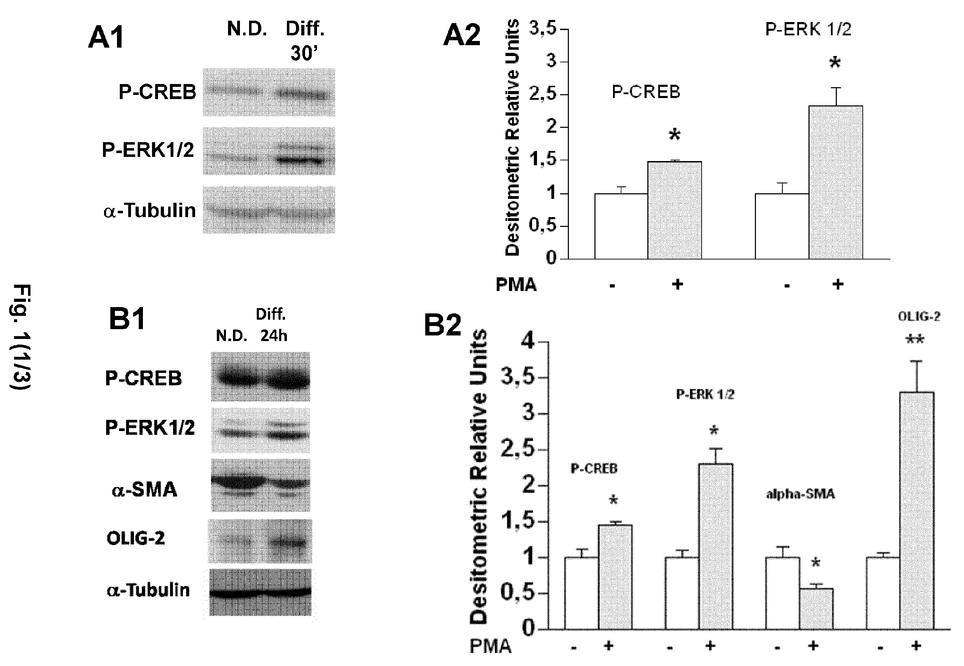

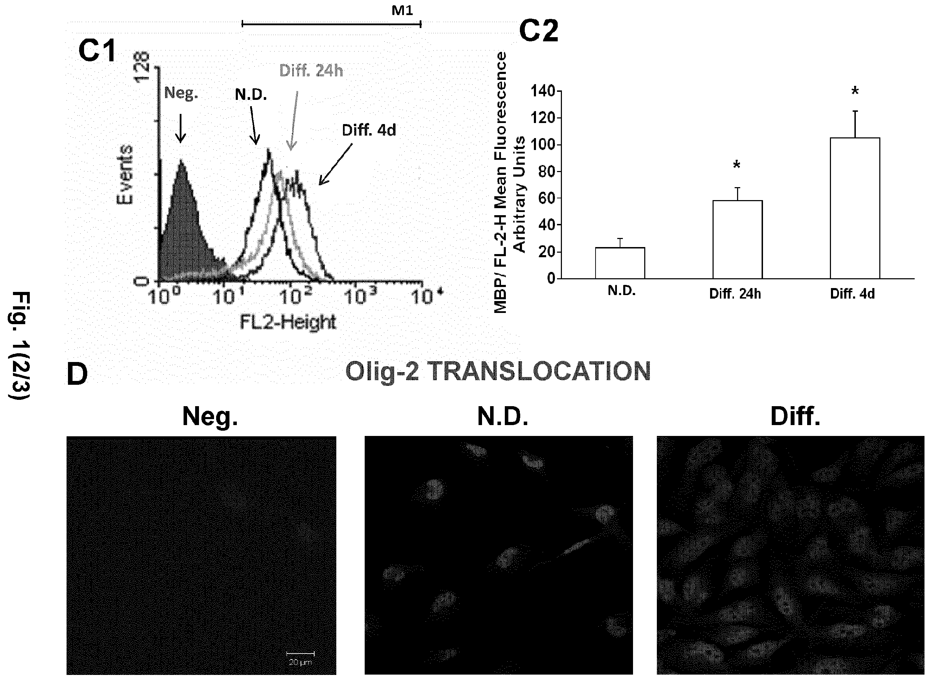

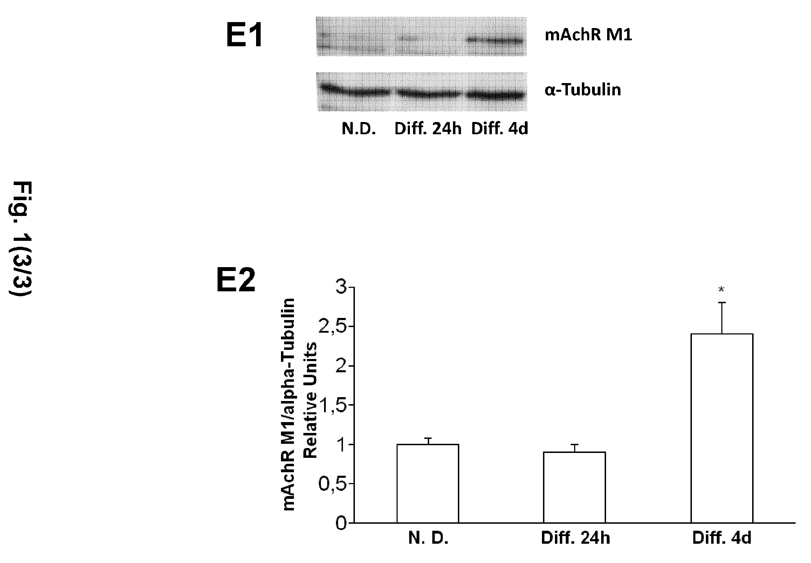

**Fig. 1****: Expression of different differentiation markers in M03-13 cells**
   **(A1)** Increase of P-CREB and P-ERK1/2 levels in M03-13 cells stimulated with PMA. The not differentiated (N.D.) cells were incubated for 16h in medium containing 0.2% of FBS and then stimulated with 100nM PMA for 30 min (Differentiated, Diff.) before harvesting them for Western blotting analysis of P-CREB and P-ERK1/2 levels. **(A2)** The histogram shows the values (means ± SEM) relative to control obtained by densitometric analysis of protein bands normalized for α-tubulin of three independent experiments. * p< 0.01 vs N.D. **(B1)** Western blotting analysis of P-CREB, P-ERK ½,α-SMA and Olig-2 levels in M03-13 cells after 24h of differentiation of M03-13 cells with 100nM PMA in medium without serum. **(B2)** The histogram shows the values (means ± SEM) relative to control obtained by densitometric analysis of protein bands normalized for α-tubulin of three independent experiments. * p< 0.01 vs N.D. **p< 0.001 vs N.D. **(C1)** MBP protein increased levels when compared to not differentiated cells (N.D.) at different days (1 day or 4 days) of differentiation (Diff.) of M03-13 cells with 100nM PMA in medium without serum. Immunoreactivity for MBP was evidenced by indirect immunofluorescence and flow cytometry using primary antibodies against MBP and CY3-conjugated anti rabbit IgG as secondary antibodies. Negative (Neg.) was treated with secondary antibodies alone. Ten thousands cells were counted for each sample. **(C2)** The histogram shows the means ± SEM of three independent experiments. N.D. indicates cells growing in complete medium. * p< 0.01 vs N.D. **(D)** Translocation of Olig-2 transcription factor from nucleus to cytosol after 24h of differentiation of M03-13 cells with 100nM PMA in medium without serum (Diff). Cells were stained with anti-human Olig-2 antibodies and CY3-conjugated anti rabbit IgG as secondary antibodies and analyzed by confocal microscopy. Neg. was treated with secondary antibodies alone. N.D. indicates cells growing in complete medium. **(E1)** mAchR M1 protein increased levels when compared to N.D. at different days of differentiation of MO3-13 cells with PMA 100nM in medium without serum. mAchR M1 levels were measured by Western blotting analysis. (**E2)** The hystogram shows the values (means ± SEM) relative to N.D., obtained by densitometric analysis of protein bands normalized for α-tubulin of three independent experiments. * p< 0.05 vs N.D.

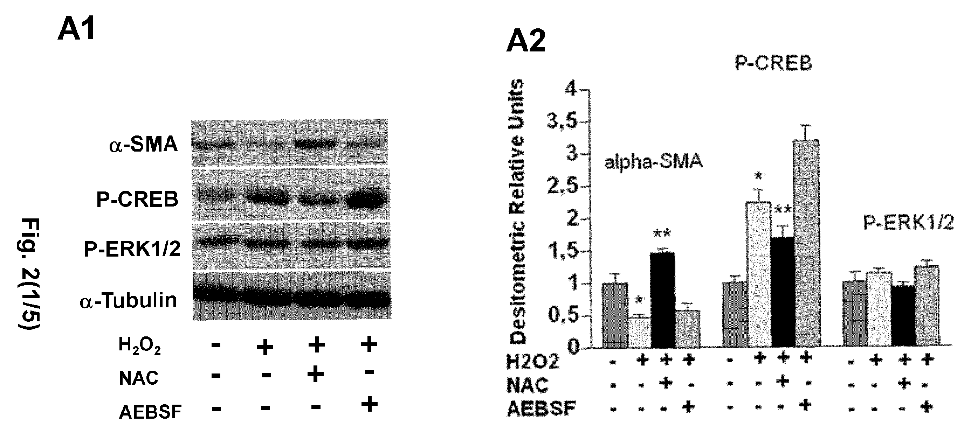

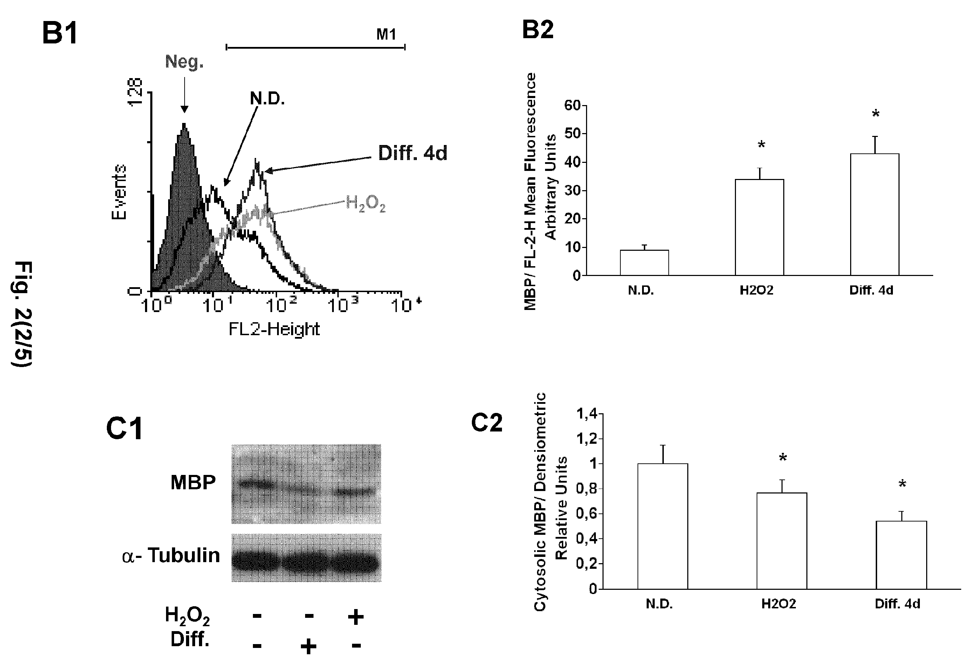

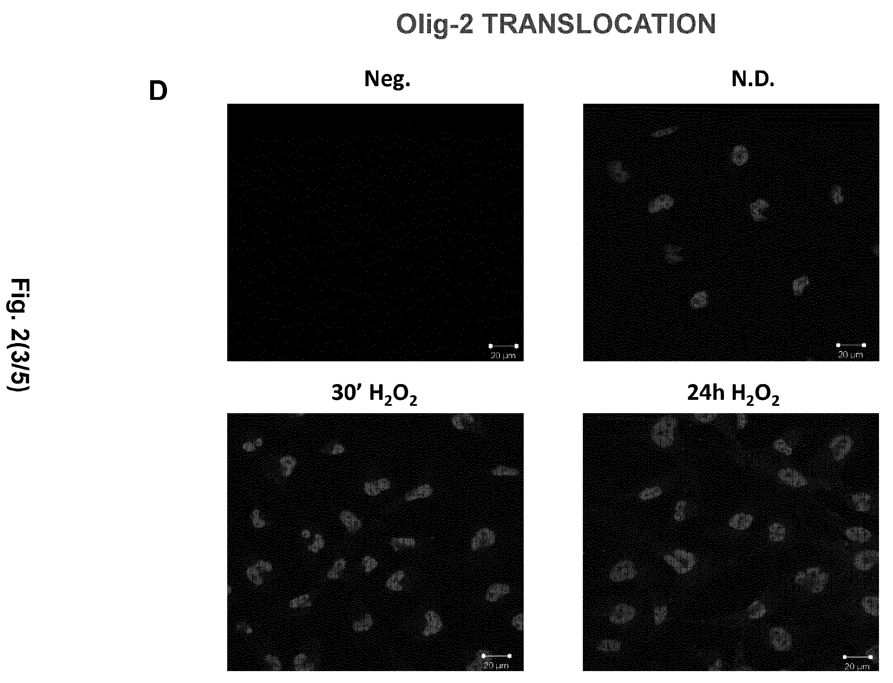

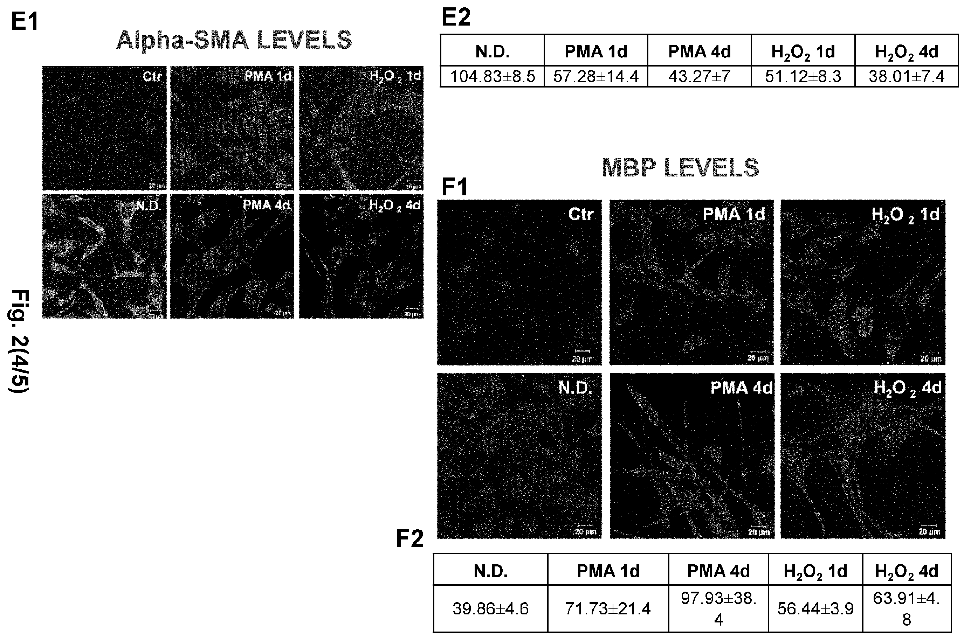

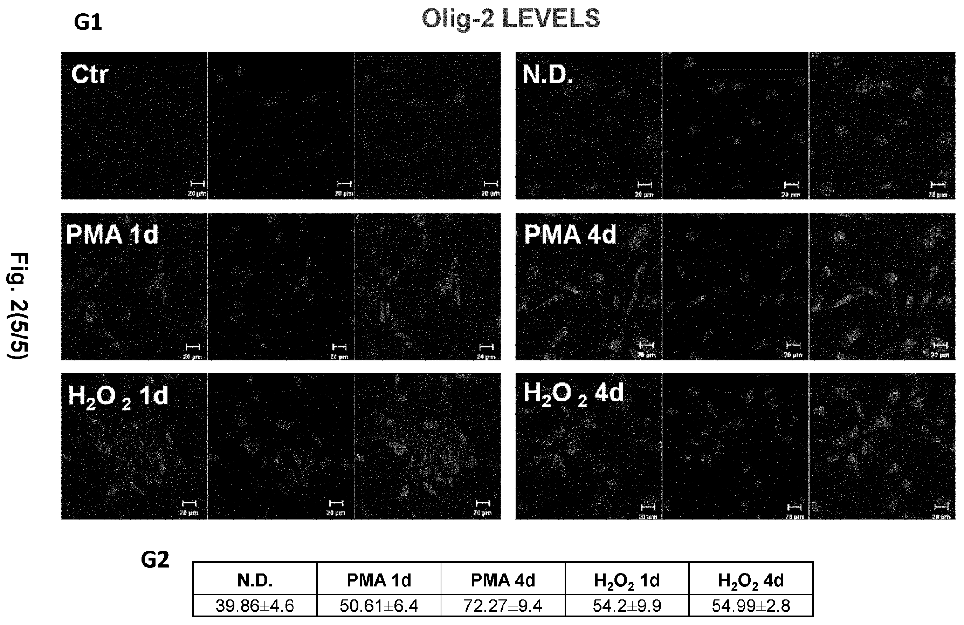

**Fig. 2****: Effects of hydrogen peroxide on differentiation markers in M03-13 cells**
   **(A1)** Western blotting analysis of α-SMA, P-CREB and P-ERK1/2 levels on M03-13 cells treated for 24h with 200µM H₂O₂ in the absence or presence of N-Acetyl-Cysteine (NAC, 10mM) or 4-(2-aminoethyl) benzene sulfonyl fluoride (AEBSF, 40µM). **(A2)** The histogram shows means ± SEM values relative to control of three independent experiments. * p< 0.01 vs control; ** p<0.01 vs. H₂O₂ treatment. **(B1)** Increase of MBP protein levels in M03-13 cells treated for 4d with 200µM H₂O₂. Immunoreactivity for MBP was evidenced by indirect immunofluorescence and flow cytometric analysis as described in Fig. 1C. A sample of cells differentiated for 4d with 100nM PMA in medium without serum is also shown (Diff. 4d). Neg. was treated with secondary antibodies alone. Ten thousands cells were counted for each sample. **(B2)** The histogram shows the mean ± SEM of three independent experiments. * p<0.01 vs N.D. **(C1)** Western blotting analysis of cytosolic MBP levels in M03-13 cells treated for 4d with 200µM H₂O₂. A sample of cells differentiated for 4d with 100nM PMA in medium without serum is also shown (Diff. 4d). **(C2)** The histogram shows the values (means ± SEM) relative to control obtained by densitometric analysis of MBP normalized for α-tubulin of three independent experiments. * p< 0.01 vs. N.D. **(D)** Translocation of Olig-2 transcription factor from nucleus to cytosol after 30min and 24h of treatment of M03-13 cells with 200µM H₂O₂. Cells were stained with anti-human Olig-2 antibodies and CY3-conjugated anti rabbit IgG as secondary antibodies and analyzed by confocal microscopy. Neg. was treated with secondary antibodies alone. N.D. indicates cells growing in complete medium. **(E1)** Decreased *α-SMA* levels in M03-13 cells after treatment with 100nM PMA in medium without serum or with 200µM H₂O₂ for 1 and 4 days. Cells were stained with anti-human *α-SMA* antibodies and CY2-conjugated anti mouse IgG as secondary antibodies and analyzed by confocal microscopy. Ctr was treated with secondary antibodies and nuclear dye DAPI alone. **(E2)** The table shows the values (mean ± SD, fluorescence arbitrary units) obtained by the quantitative analysis of 25 cells for each sample. **(F1)** Increased *MBP* levels in M03-13 cells after treatment with 100nM PMA in medium without serum or with 200µM H₂O₂ for 1 and 4 days. Cells were stained with anti-human *MBP* antibodies and CY3-conjugated anti rabbit IgG as secondary antibodies and analyzed by confocal microscopy. Ctr was treated with secondary antibodies and nuclear dye DAPI alone. **(F2)** The table shows the values (mean ± SD, fluorescence arbitrary units) obtained by the quantitative analysis of 25 cells for each sample. **(G1)** Increased *Olig-2* levelsin M03-13 cells after treatment with 100nM PMA in medium without serum or with 200µM H₂O₂ for 1 and 4 days.Cells were stained with anti-human *Olig-*2 antibodies and CY3-conjugated anti rabbit IgG as secondary antibodies and analyzed by confocal microscopy. For each image are shown three panels: on the left *Olig-2* (red); on the center nuclei (blue); on the right the merged image. Ctr was treated with secondary antibodies and nuclear dye DAPI alone. **(G2)** The table shows the values (mean ± SD, fluorescence arbitrary units) obtained by the quantitative analysis of 25 cells for each sample.

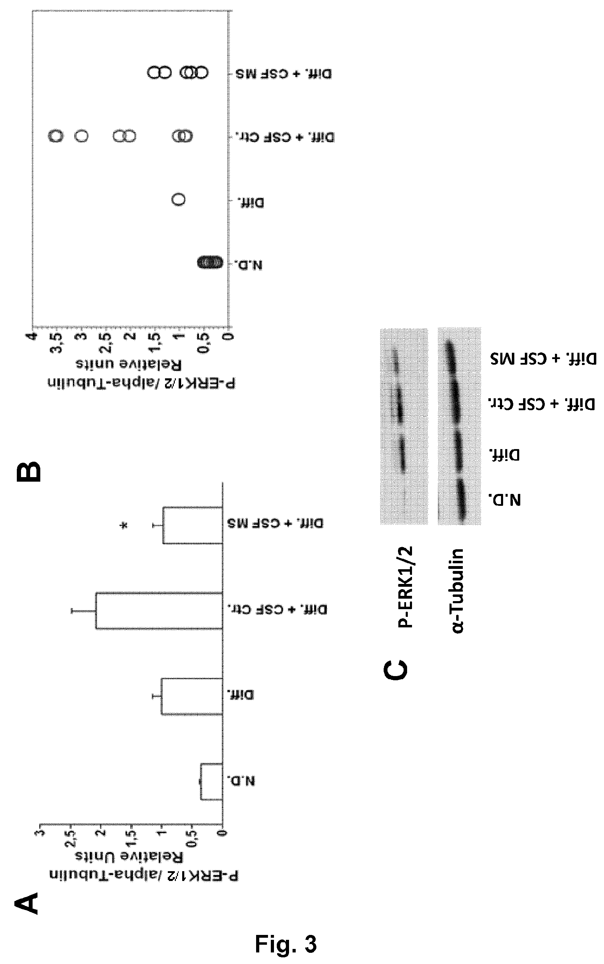

**Fig. 3****: Differential effects of CSF from Control or MS patients on P-ERK1/2 protein levels in M03-13 cells differentiated with PMA (Example 1)**
   Cells were treated for 24h with 100nM PMA in medium without serum in absence (Diff.) and presence of 30% CSF from Control (Ctr.) or multiple sclerosis (MS) patients and then harvested for Western blotting analysis of P-ERK1/2 levels. The histogram **(A)** shows the mean ± SEM of P-ERK1/2 values relative to Diff. samples obtained by densitometric analysis of P-ERK1/2 band normalized for α-tubulin. **(B)** the scatter plot with the single values for each patient is shown. **(C)** a representative Western blotting experiment is shown. * p< 0.05 vs CSF Ctr.

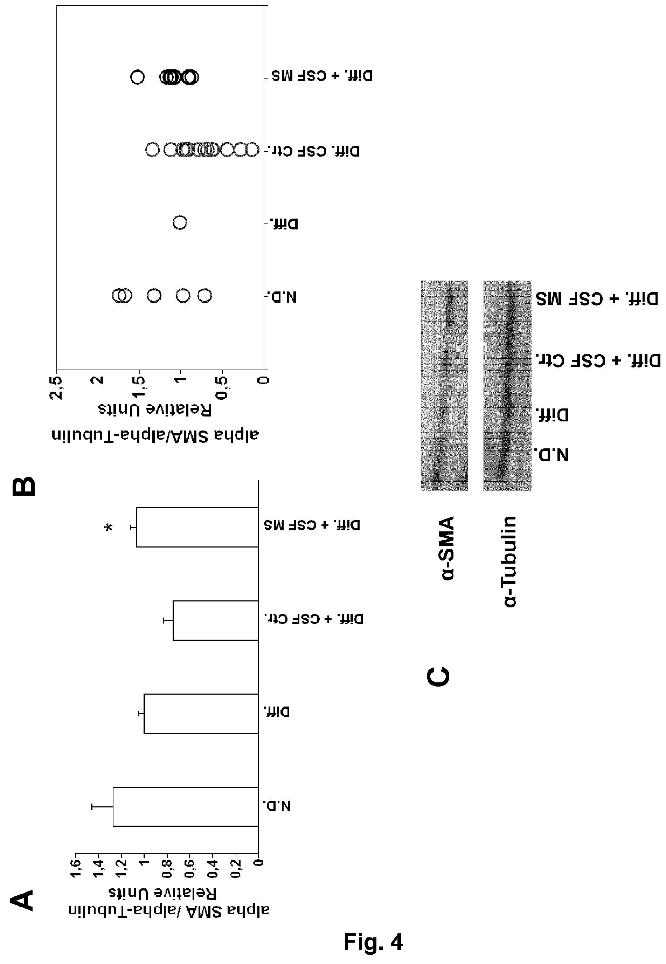

**Fig. 4****: Differential effects of CSF from Control or MS patients on α-SMA protein levels of M03-13 cells differentiated with PMA (Example 1)**
   Cells were treated for 24h with 100nM PMA in medium without serum in absence (Diff.) and presence of 30% CSF from Ctr. or MS patients and then harvested for Western blotting analysis of α-SMA levels. The histogram **(A)** shows the mean ± SEM of α-SMA values relative to Diff. samples obtained by densitometric analysis of α-SMA band normalized for α-tubulin. **(B)** the scatter plot with the single values for each patient is shown. **(C)** a representative Western blotting experiment is shown. * p< 0.05 vs CSF Ctr.

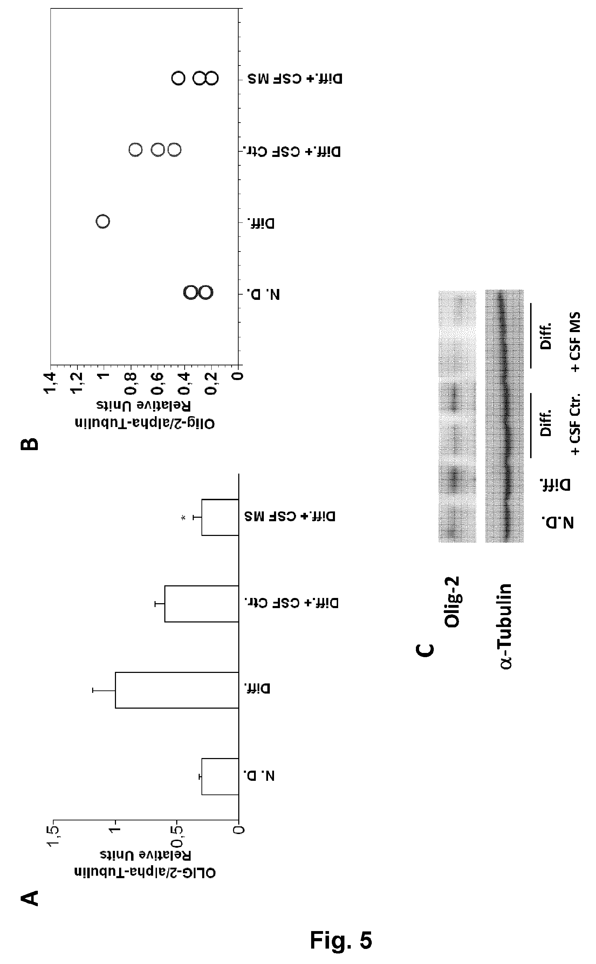

**Fig. 5****: Differential effects of CSF from Control or MS patients on Olig-2 protein levels in M03-13 cells differentiated with PMA (Example 1)**
   Cells were treated for 24h with 100nM PMA in medium without serum in absence (Diff.) and presence of 30% CSF from Control (Ctr.) or multiple sclerosis (MS) patients and then harvested for Western blotting analysis of Olig-2 levels. The histogram **(A)** shows the mean ± SEM of Olig-2 values relative to Diff. samples obtained by densitometric analysis of Olig-2 band normalized for α-tubulin. **(B)** the scatter plot with the single values for each patient is shown. **(C)** a representative Western blotting experiment is shown. *p< 0.05 vs. CSF Ctr.

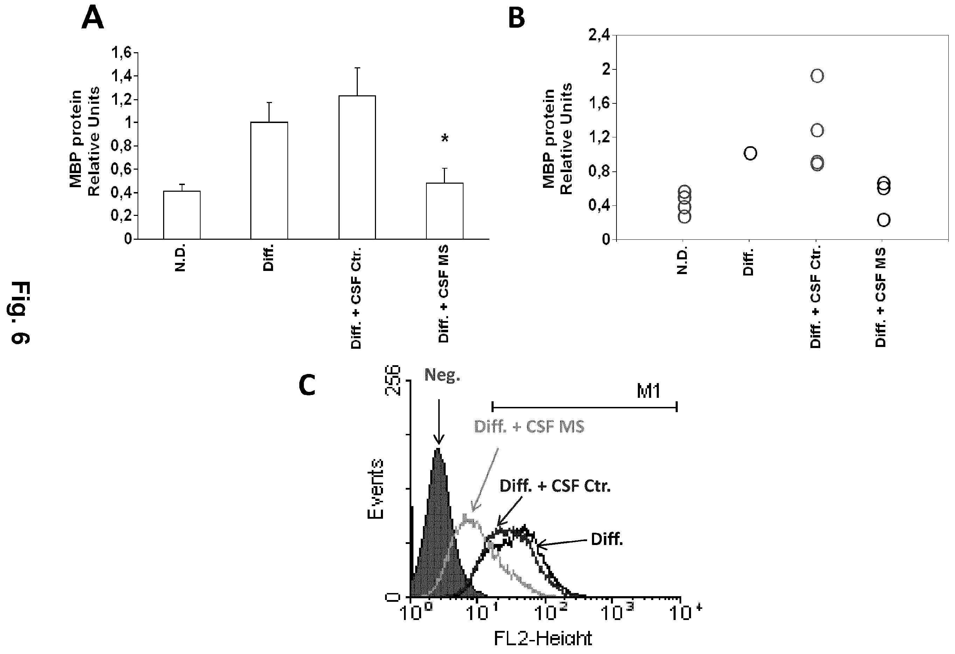

**Fig. 6****: Differential effects of CSF from Control or MS patients on MBP protein levels in M03-13 cells differentiated with PMA (Example 1)**
   **(A)** Cells were treated for 24h with 100nM PMA in medium without serum in absence (Diff.) and presence of 30% CSF from Ctr. or MS patients and then immune reactivity for MBP was evidenced by indirect immunofluorescence and flow cytometry using primary antibodies against MBP and CY3-conjugated anti rabbit IgG as secondary antibodies. Neg. was treated with secondary antibodies alone. Ten thousands cells were counted for each sample. The histogram **(A)** shows the means ± SEM fluorescence values relative to not stimulated samples; **(B)** the scatter plot with the single relative values for each patient is shown. * p< 0.05 vs CSF Ctr. The panel **(C)** shows the flow cytometric histograms of a representative experiment.

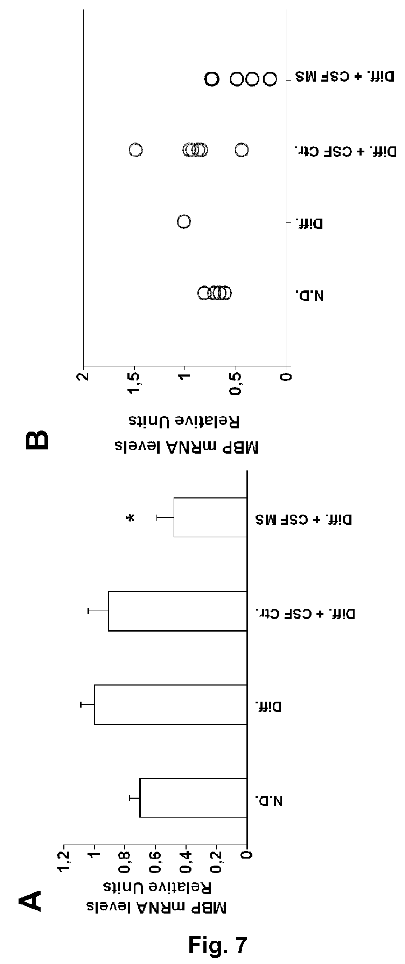

**Fig. 7****: Differential effects of CSF from Control or MS patients on MBP mRNA levels in M03-13 cells differentiated with PMA (Example 1)**
   Cells were treated for 24h with 100nM PMA in medium without serum in absence (Diff.) and presence of 30% CSF from Ctr. or MS patients and then MBP mRNA levels were analysed by RT- PCR. The histogram **(A)** shows the mean ± SEM values relative to Diff. sample. **(B)** the scatter plot with the single values for each patient. * p< 0.05 vs CSF Ctr.

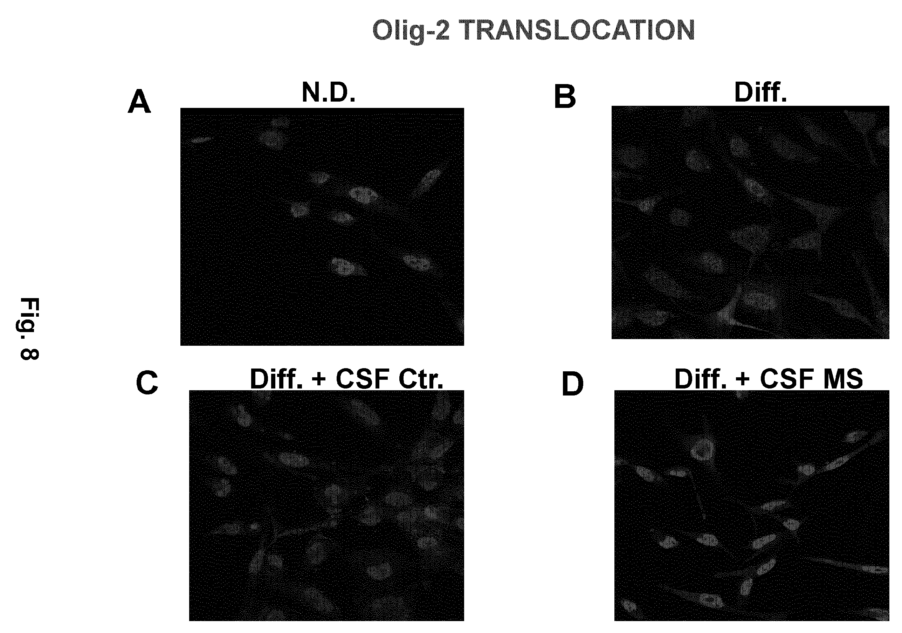

**Fig. 8****: Differential effects of CSF from Control or MS patients on translocation of OLIG-2 from nucleus to cytosol in M03-13 cells differentiated with PMA (Example 1)**
   The staining for Olig-2 in N.D. cells is shown in panel **(A).** Cells were treated for 24h with 100nM PMA in medium without serum in absence **(B)** (Diff. 24h) and presence of 30% CSF from Ctr. **(C)** or MS **(D)** patients and then stained with anti-human Olig-2 antibodies and CY3-conjugated anti rabbit IgG as secondary antibodies and analyzed by confocal microscopy.

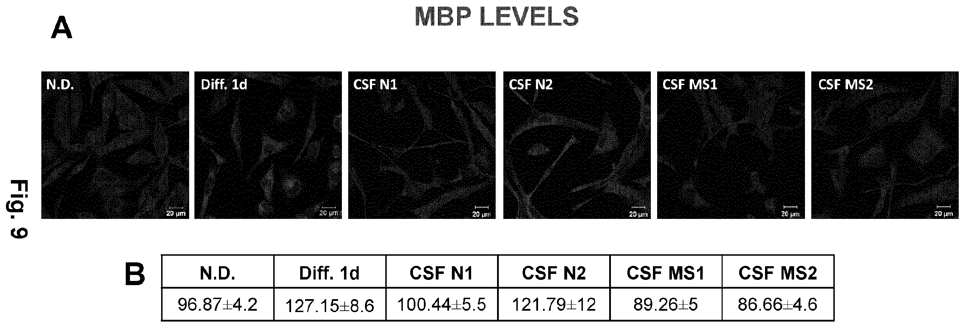

**Fig. 9****: CSF from MS patients inhibits PMA differentiative effect on *MBP* levels after 1d (Example 1)**
   **(A)** The cells were stained with anti*-MBP* and anti-rabbit secondary antibody conjugated to CY3 and the nuclear dye DAPI, and analyzed by confocal microscopy. Diff. was treated only with PMA 100 nM in serum-free medium for 1 day; CSF N indicates cells stimulated by CSF from control patients; CSF MS indicates cells stimulated with CSF of patients with MS. **(B)** The table shows the values (mean ± SD, fluorescence arbitrary units) obtained by the quantitative analysis of 25 cells for each sample.

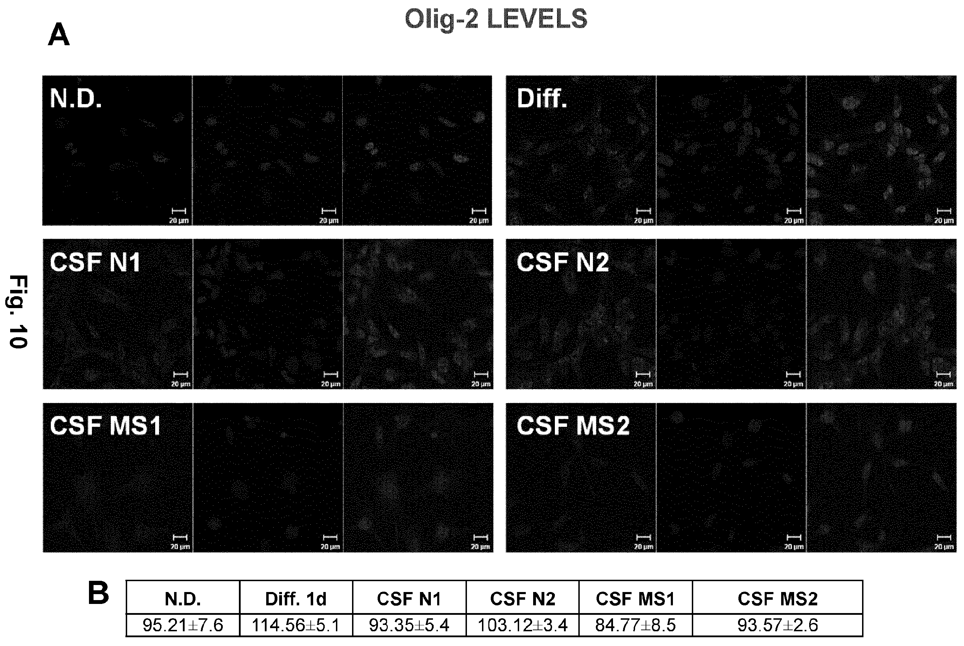

**Fig. 10****: CSF from MS patients inhibits PMA differentiative effect on *Olig-2* levels after 1d (Example 1)**
   **(A)** The cells were stained with anti-*Olig-2* and anti-rabbit secondary antibody conjugated to CY3 and the nuclear dye DAPI, and analyzed by confocal microscopy.For each image are shown three panels: on the left *Olig-2* (red); on the center nuclei (blue); on the right the merged image. Diff. was treated only with PMA 100 nM in serum-free medium for 1 day; CSFN indicates cells stimulated by CSF from control patients; CSFMS indicates cells stimulated with CSF of patients with MS. **(B)** The table shows the values (mean ± SD, fluorescence arbitrary units) obtained by the quantitative analysis of 25 cells for each sample.

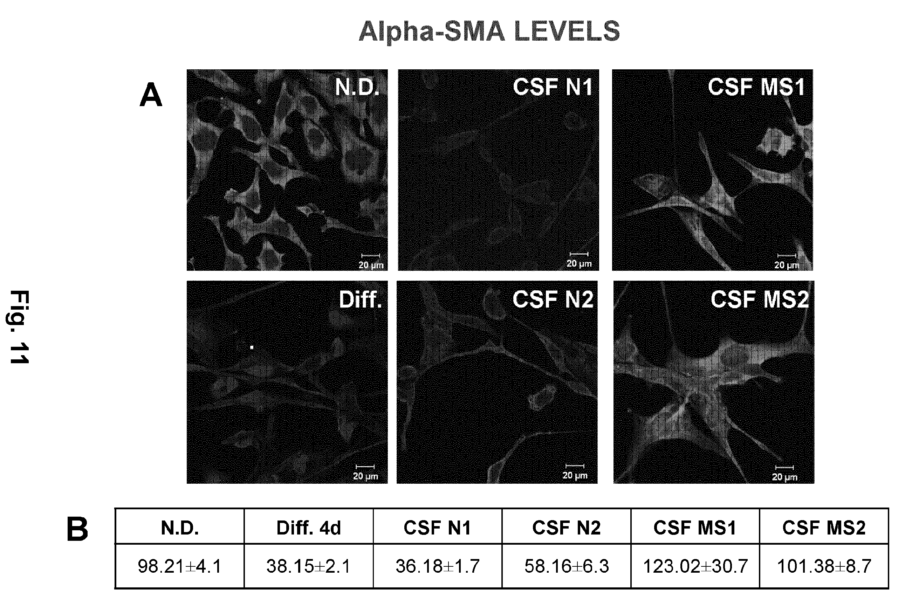

**Fig. 11****: CSF from MS patients inhibits PMA differentiative effect on *α-SMA* levels after 4 d (Example 1)**
   **(A)** The cells were stained with anti*-α-SMA* and anti-mouse secondary antibody conjugated to CY2 and the nuclear dye DAPI, and analyzed by confocal microscopy. Diff was treated only with PMA100 nM in serum-free medium for 4 days; CSFN indicates cells stimulated by CSF from control patients; CSFMS indicates cells stimulated with CSF of patients with MS. **(B)** The table shows the values (mean ± SD, fluorescence arbitrary units) obtained by the quantitative analysis of 25 cells for each sample.

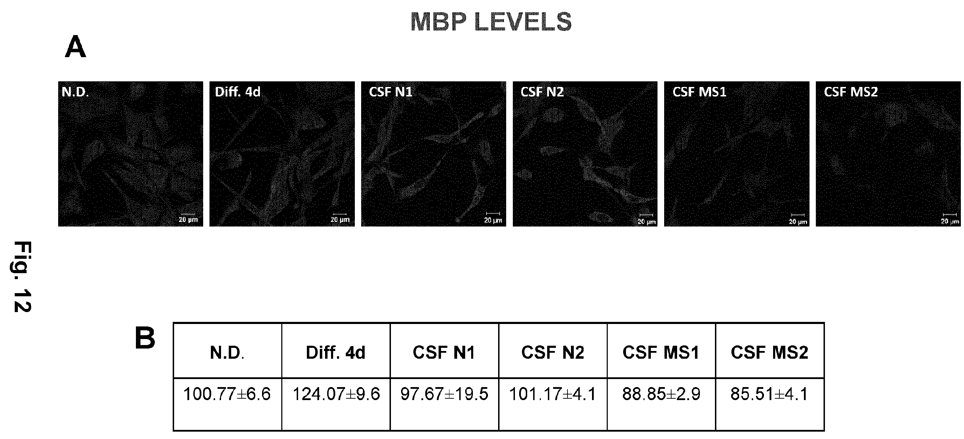

**Fig. 12****: CSF from MS patients inhibits PMA differentiative effect on *MBP* levels after 4d (Example 1)**
   **(A)** The cells were stained with anti-*MBP* and anti-rabbit secondary antibody conjugated to CY3 and the nuclear dye DAPI, and analyzed by confocal microscopy. Diff was treated only with PMA 100 nM in serum-free medium for 4 days; CSFN indicates cells stimulated by CSF from control patients; CSFMS indicates cells stimulated with CSF of patients with MS. **(B)** the table shows the fluorescence values (mean ± SD, arbitrary units) obtained by the quantitative analysis of 25 cells for each sample.

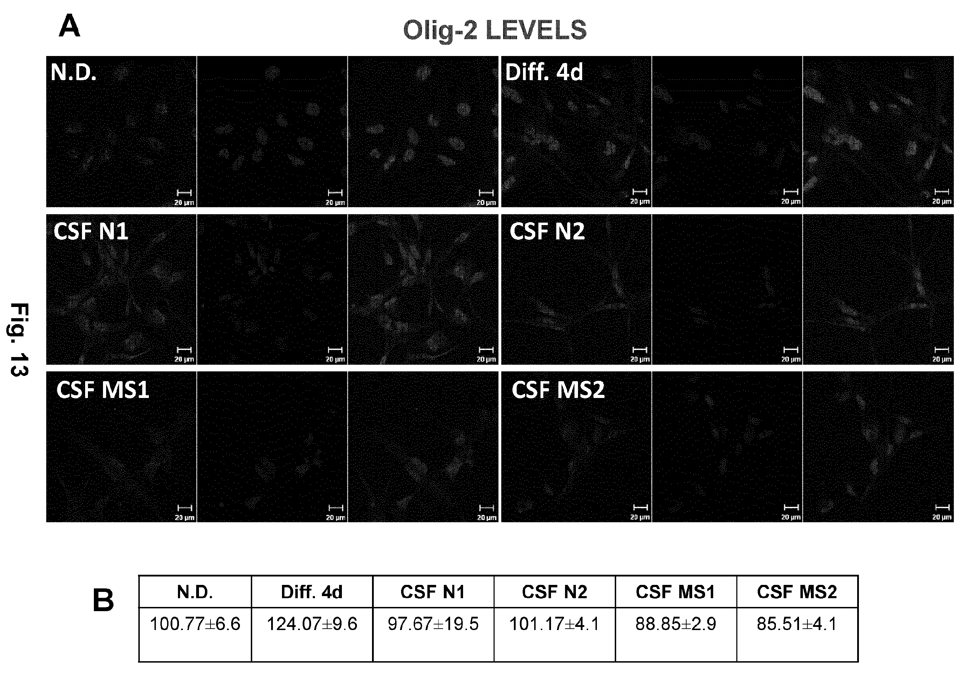

**Fig. 13****: CSF from MS patients inhibits PMA differentiative effect on *Olig-2* levels after 4d (Example 1)**
   (A) The cells were stained with anti-*Olig-2* and anti-rabbit secondary antibody conjugated to CY3and the nuclear dye DAPI, and analyzed by confocal microscopy.For each image are shown three panels: on the left *Olig-2* (red); on the center nuclei (blue); on the right the merged image. Diff. was treated only with PMA100nM in serum-free medium for 4 days; CSFN indicates cells stimulated by CSF from control patients; CSFMS indicates cells stimulated with CSF of patients with MS. **(B)** The table shows the values (mean ± SD, fluorescence arbitrary units) obtained by the quantitative analysis of 25 cells for each sample.

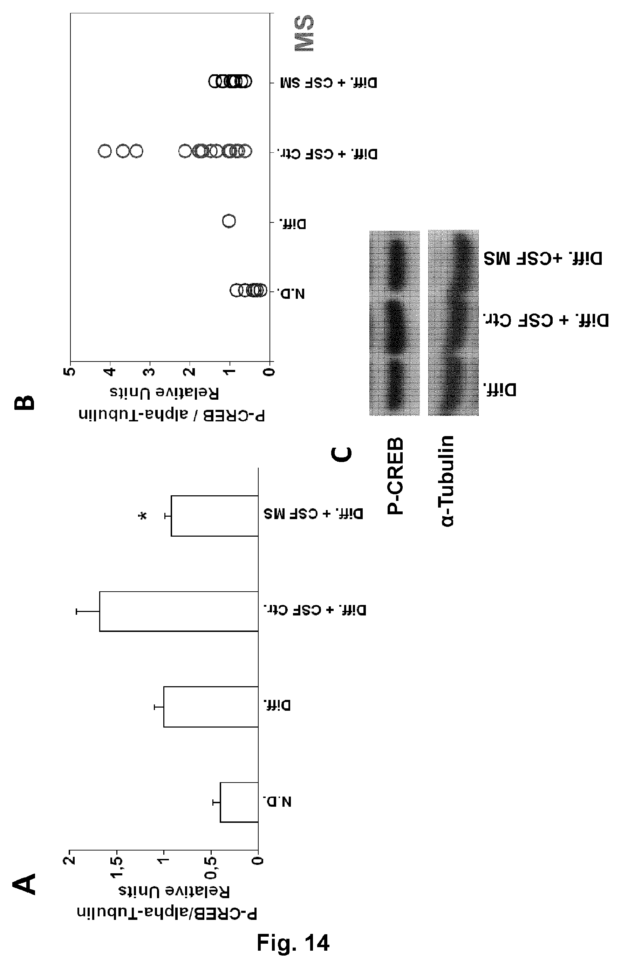

**Fig. 14****: Differential effects of 30 min exposure to CSF from Control or MS patients on P-CREB levels in differentiated M03-13 (Example 2)**
   Cells were differentiated for 4 days with 100nM PMA in medium without serum, stimulated for 30 min with 30% CSF from Ctr. or MS patients and then P-CREB levels were evaluated by Western blotting analysis. The histogram **(A)** shows the mean ± SEM levels relative to cells not stimulated with CSF (Diff.), obtained by densitometric analysis of protein bands normalized for α-tubulin. **(B)** the scatter plot with the single values for each patient. **(C)** a representative Western blotting experiment is shown. * p< 0.05 vs CSF Ctr.

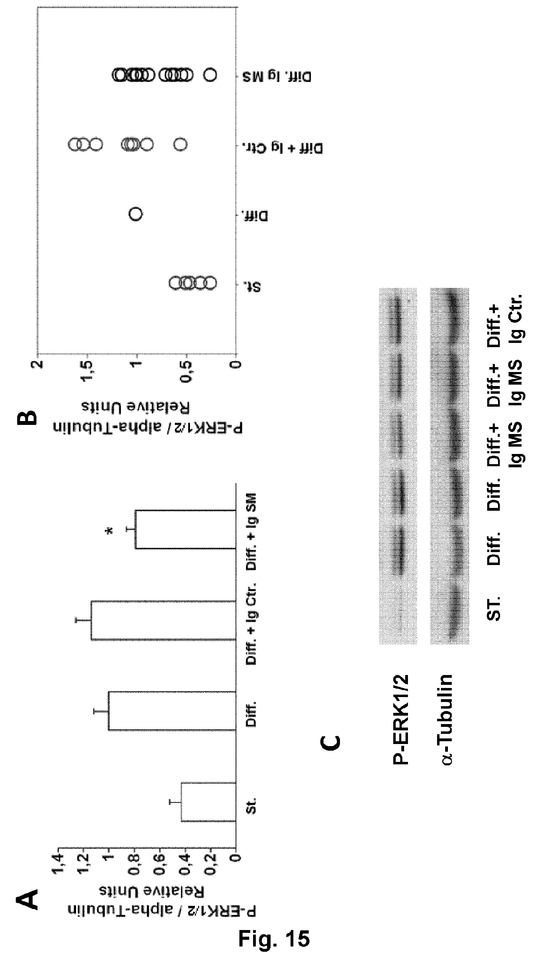

**Fig. 15****: Differential effects of 30 min exposure to serum IgG from Control or MS patients on P-ERK1/2 levels in M03-13 (Example 3)**
   Cells were cultured in medium containing 0.2% FBS for 16h, preincubated for 30 min in the absence (Diff.) or presence of 200 µg/ml of serum IgG and then stimulated with 100 nM PMA for 30 min before harvesting them for Western blotting analysis of P-ERK1/2 levels. The histogram **(A)** shows the mean ± SEM of P-ERK1/2 values relative to Diff. sample obtained by densitometric analysis of protein bands normalized for α-tubulin. **(B)** scatter plot with the single values for each patient is shown. **(C)** a representative Western blotting experiment is shown. * p< 0.05 vs Ig Ctr.

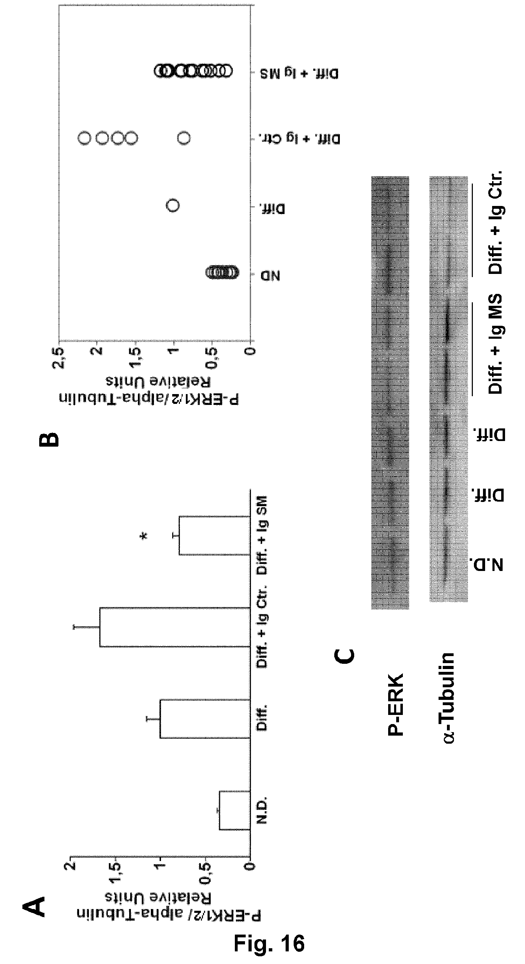

**Fig. 16****: Differential effects of serum IgG from Control or MS patients on P-ERK1/2 protein levels in M03-13 cells differentiated with PMA (Example 3)**
   Cells were treated for 24h with 100 nM PMA in medium without serum in the absence (Diff.) or presence of 200 µg/ml of serum IgG from Ctr. or MS patients and then P-ERK1/2 levels were evaluated by Western blotting analysis. The histogram **(A)** shows the mean ± SEM of P-ERK1/2 values relative to Diff. samples obtained by densitometric analysis of P-ERK1/2 band normalized for α-tubulin. **(B)** scatter plot with the single values for each patient is shown. **(C)** a representative Western blotting experiment is shown. *p< 0.05 vs Ig Ctr.

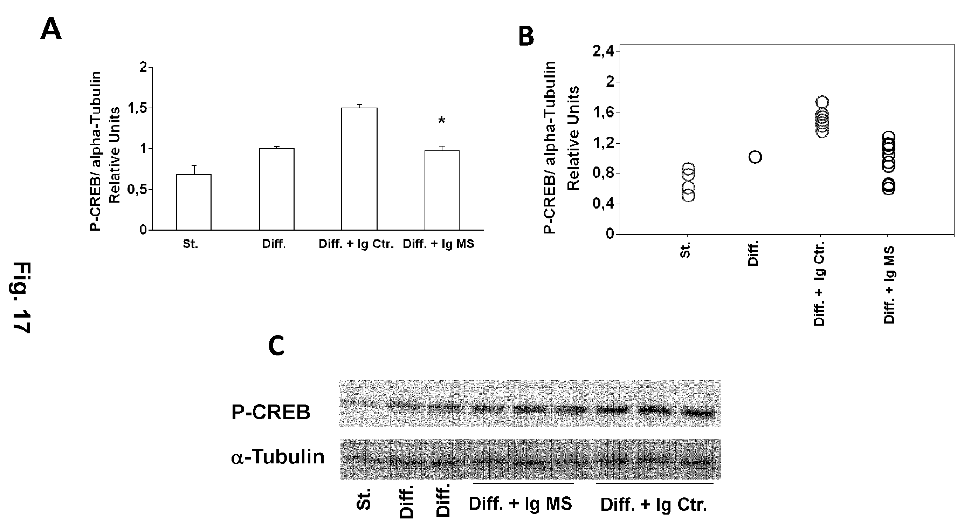

**Fig. 17****: Differential effects of 30 min exposure to serum IgG from Normal or MS patients on P-CREB levels in M03-13 (Example 4)**
   Cells were treated as in Fig. 15 and then P-CREB levels were measured by Western blotting analysis. The histogram **(A)** shows the mean ± SEM of P-CREB values relative to Diff. sample obtained by densitometric analysis of protein bands normalized for α-tubulin. **(B)** scatter plot with the single values for each patient is shown. **(C)** a representative Western blotting experiment is shown. *p< 0.05 vs Ig Ctr.

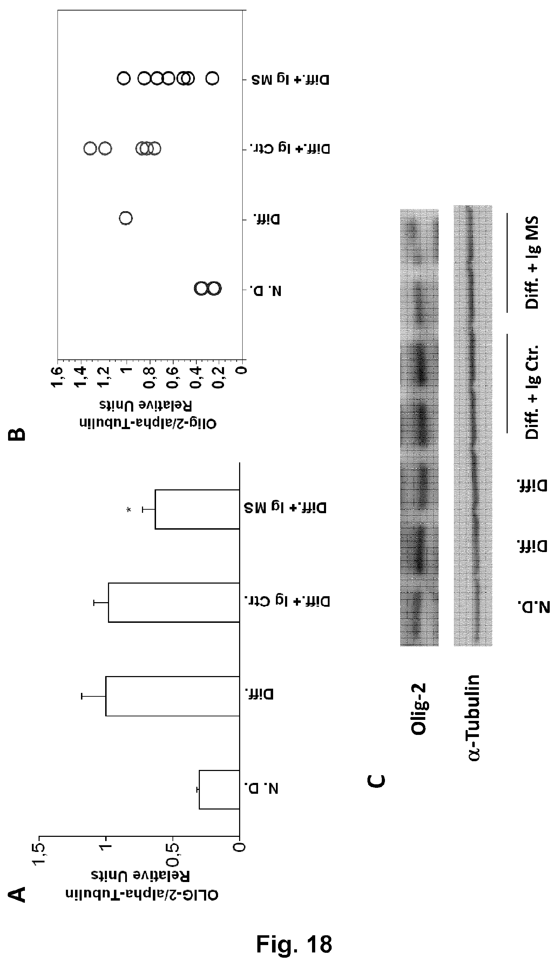

**Fig. 18****: Differential effects of serum IgG from Control or MS patients on olig-2 protein levels in M03-13 cells differentiated with PMA (Example 5)**
   Cells were treated as in Fig. 16 and then olig-2 protein levels were measured by Western blotting. The histogram **(A)** shows the mean ± SEM of olig-2 values relative to Diff. samples obtained by densitometric analysis of olig-2 protein band normalized for α-tubulin. **(B)** scatter plot with the single values for each patient is shown. **(C)** a representative Western blotting experiment is shown. *p< 0.05 vs Ig Ctr.

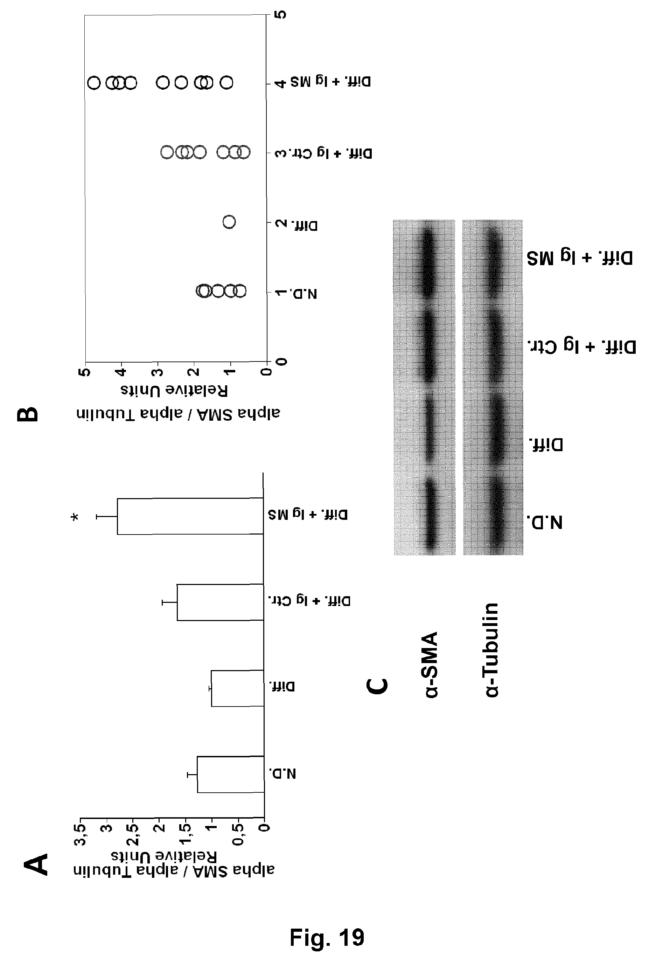

**Fig. 19****: Differential effects of serum IgG from Control or MS patients on α-SMA protein levels in M03-13 cells differentiated with PMA (Example 6)**
   Cells were treated as in Fig. 16 and then α-SMA levels were evaluated by Western blotting analysis. The histogram **(A)** shows the mean ± SEM of α-SMA values relative to Diff. samples obtained by densitometric analysis of α-SMA band normalized for α-tubulin. **(B)** scatter plot with the single values for each patient is shown. **(C)** a representative Western blotting experiment is shown. *p< 0.05 vs Ig Ctr.

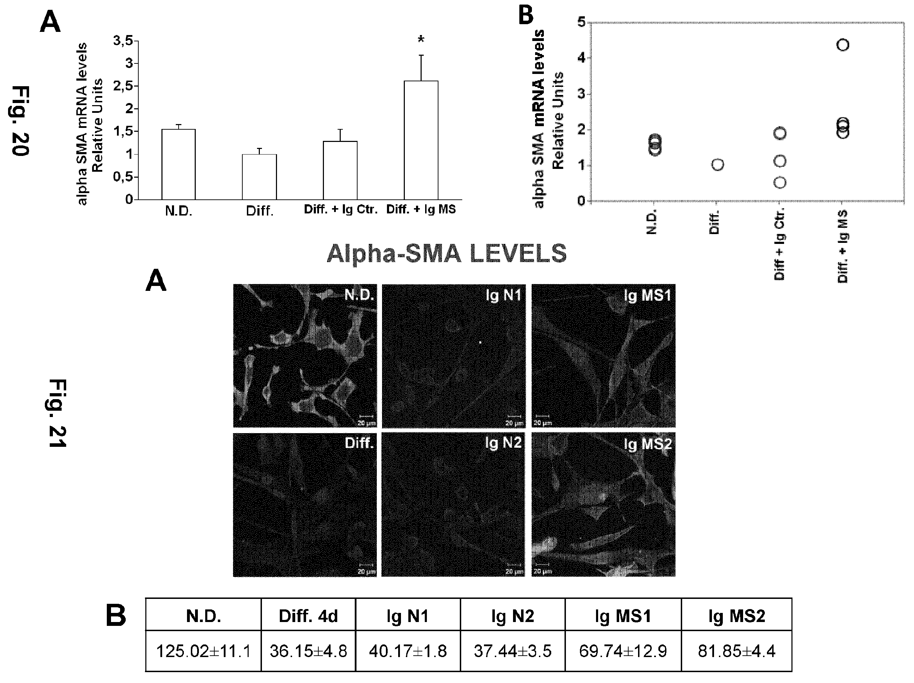

**Fig. 20****: Differential effects of serum IgG from control or MS patients on α-SMA mRNA levels in M03-13 cells differentiated with PMA (Example 6)**
   Cells were treated for 24h with 100nM PMA in medium without serum in absence (Diff.) and presence 200 µg/ml of serum IgG from Ctr. or MS patients and then α-SMA mRNA levels were evaluated by RT-PCR analysis. The histogram **(A)** shows the mean ± SEM values relative to Diff. samples. **(B)** scatter plot with the single values for each patient. *p< 0.05 vs Ig Ctr.
**Fig. 21****: IgG from MS patients inhibit PMA differentiative effect on *α-SMA* levels after 4d (Example 6)**
   **(A)** The cells were stained with anti-*α-SMA* and anti-mouse secondary antibody conjugated to CY2 and the nuclear dye DAPI, and analyzed by confocal microscopy. Diff. was treated only with PMA 100nM in serum-free medium for 4 days; Ig N indicates cells stimulated by IgG of control patients; Ig MS indicates cells stimulated with IgG of patients with MS. **(B)** The table shows the values (mean ± SD, fluorescence arbitrary units) obtained by the quantitative analysis of 25 cells for each sample.

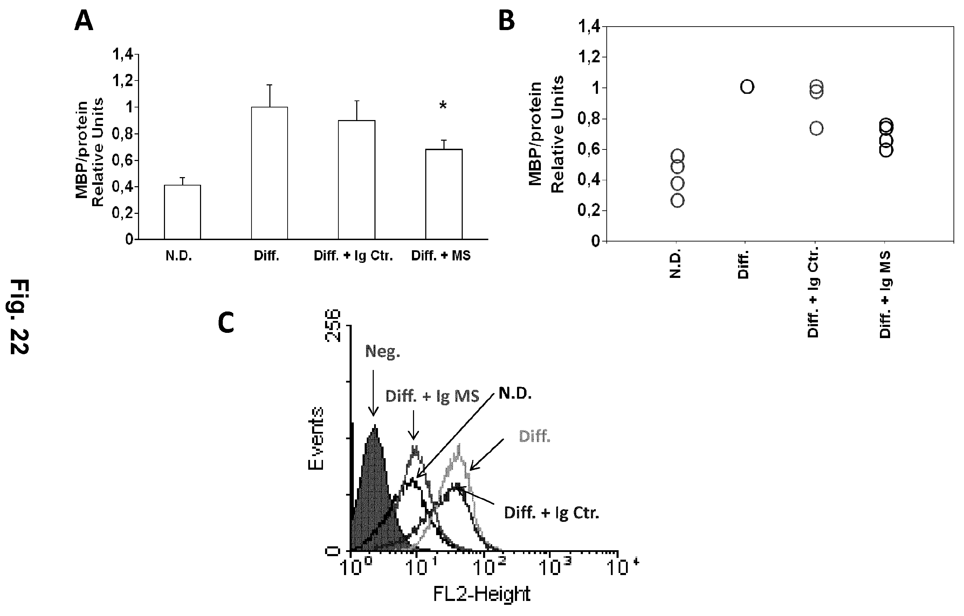

**Fig. 22****: Differential effects of serum IgG from Control or MS patients on total MBP protein levels in M03-13 cells differentiated with PMA (Example 7)**
   Cells were treated as in Fig. 16 and then immunoreactivity for MBP was evidenced by indirect immunofluorescence and flow cytometry using primary antibodies against MBP and CY3-conjugated anti rabbit IgG as secondary antibodies. Neg. was treated with secondary antibodies alone. Ten thousands cells were counted for each sample. The histogram **(A)** shows the means ± SEM values relative to Diff. sample; **(B)** scatter plot with the single relative values for each patient is shown. **(C)** flowcytometric histograms of a representative experiment are shown. *p< 0.05 vs Ig Ctr.

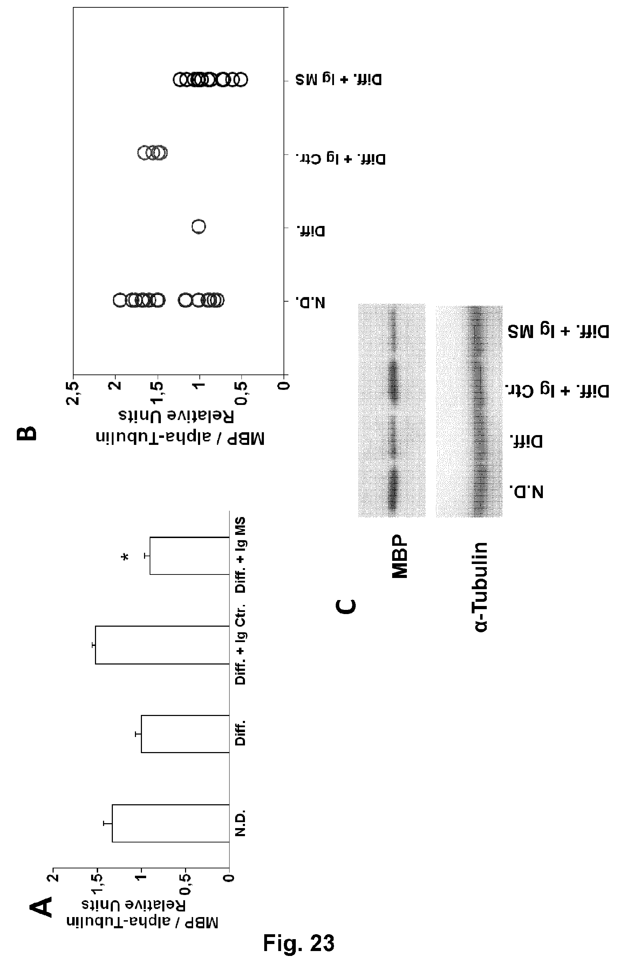

**Fig. 23****: Differential effects of serum IgG from Control or MS patients on cytosolic MBP protein levels in M03-13 cells differentiated with PMA (Example 7)**
   Cells were treated as in Fig. 16 and then cytosolic MBP protein levels were measured by Western blotting. The histogram **(A)** shows the mean ± SEM of cytosolic MBP values relative to Diff. samples obtained by densitometric analysis of MBP protein band normalized for α-tubulin. **(B)** scatter plot with the single values for each patient is shown. **(C)** a representative Western blotting experiment is shown. *p< 0.05 vs Ig Ctr.

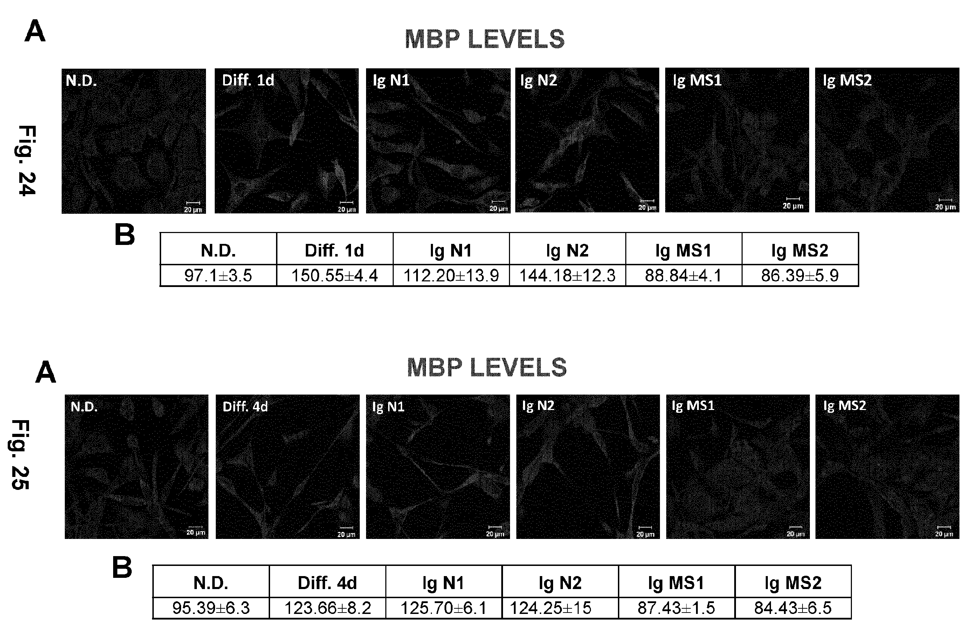

**Fig. 24****: IgG from MS patients inhibit PMA differentiative effect on *MBP* levels after 1 d (Example 7)**
   **(A)** The cells were stained with anti *MBP* and anti-rabbit secondary antibody conjugated to CY3and the nuclear dye DAPI, and analyzed by confocal microscopy. Diff. was treated only with PMA 100 nM in serum-free medium for 1 day; Ig N indicates cells stimulated by IgG of control patients; Ig MS indicates cells stimulated with IgG of patients with MS. **(B)** The table shows the fluorescence values (mean ± SD, arbitrary units) obtained by the quantitative analysis of 25 cells for each sample.
**Fig. 25****: IgG from MS patients inhibit PMA differentiative effect on *MBP* levels after 4 d (Example 7)**
   **(A)** The cells were stained with anti-*MBP* and anti-rabbit secondary antibody conjugated to CY3 and the nuclear dye DAPI, and analyzed by confocal microscopy. Diff. was treated only with PMA100 nM in serum-free medium for 4 days; Ig N indicates cells stimulated by IgG of control patients; Ig MS indicates cells stimulated with IgG of patients with MS. **(B)** The table shows the fluorescence values (mean ± SD, arbitrary units) obtained by the quantitative analysis of 25 cells for each sample.
**Fig. 26****: Differential effects of serum IgG from Control or MS patients on translocation of Olig-2 from nucleus to cytosol in M03-13 cells differentiated with PMA (Example 8)**
   Cells were treated for 24h with 100nM PMA in medium without serum in the absence or presence of 200µg/ml of serum IgG from Control or MS patients and then stained with Olig-2 antibodies and analyzed by confocal microscopy. The staining for Olig-2 in N.D. cells is also shown.
**Fig. 27****: IgG from MS patients inhibit PMA differentiative effect on *Olig-2* levels after 1d (Example 8)**
   **(A)** The cells were stained with anti-*Olig-2* and anti-rabbit secondary antibody conjugated to CY3and the nuclear dye DAPI, and analyzed by confocal microscopy. For each image are shown three panels: on the left *Olig-2* (red); on the center nuclei (blue); on the right the merged image. Diff. was treated only with PMA 100 nM in serum-free medium for 1 day; Ig N indicates cells stimulated by IgG of control patients; Ig MS indicates cells stimulated with IgG of patients with MS. **(B)** The table shows the values (mean ± SD, fluorescence arbitrary units) obtained by the quantitative analysis of 25 cells for each sample.

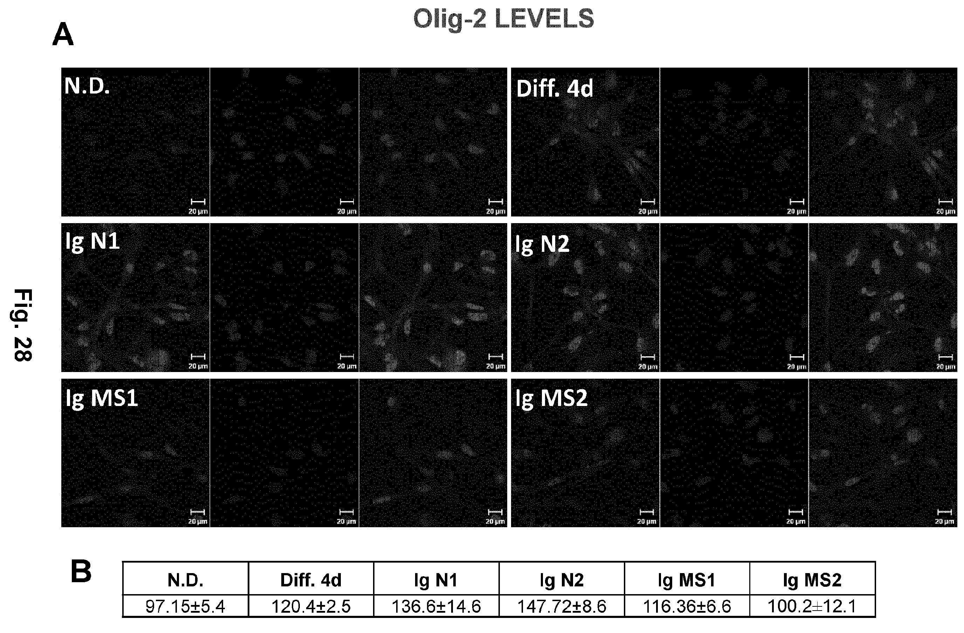

**Fig. 28****: IgG from MS patients inhibit PMA differentiative effect on *Olig-2* levels after 4d (Example 8)**
   **(A)** The cells were stained with anti-*Olig-2* and anti-rabbit secondary antibody conjugated to CY3 and the nuclear dye DAPI, and analyzed by confocal microscopy. For each image are shown three panels: on the left *Olig-2* (red); on the center nuclei (blue); on the right the merged image. Diff. was treated only withPMA100 nM in serum-free medium for 4 days; Ig N indicates cells stimulated by IgG of control patients; Ig MS indicates cells stimulated with IgG of patients with MS. **(B)** The table shows the values (mean ±SD, fluorescence arbitrary units) obtained by the quantitative analysis of 25 cells for each sample.

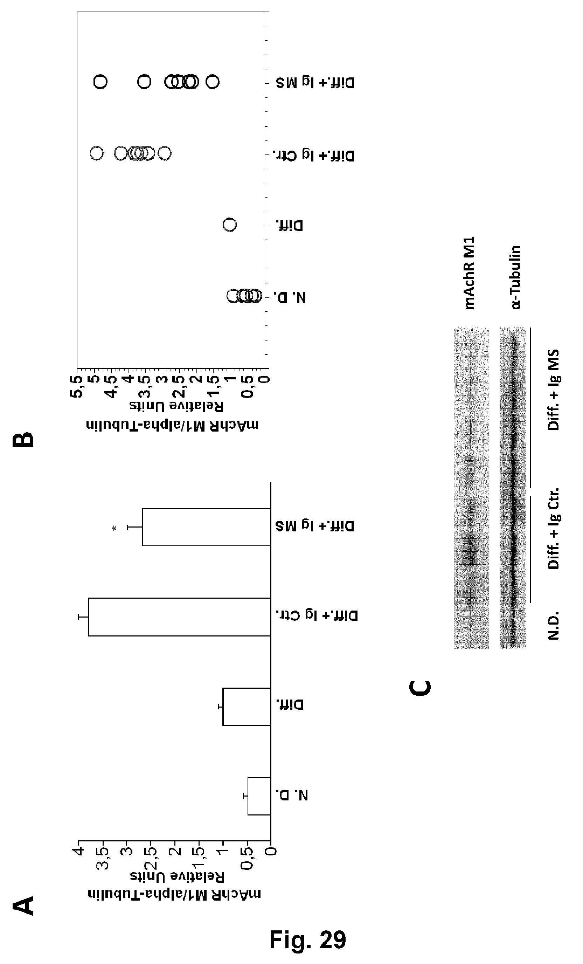

**Fig. 29****: Differential effects of serum IgG from Control or MS patients on mAchR M1 protein levels in MO3-13 cells differentiated with PMA for 4 d (Example 9)**
   Cells were treated for 4 days with 100nM PMA in medium without serum in the absence (Diff.) or presence of 200µM of serum IgG from Ctr. or MS patients and then mAchR M1 levels were evaluated by Western blotting analysis. The histogram **(A)** shows the mean ± SEM of mAchR M1 levels relative to Diff. samples, obtained by densitometric analysis of mAchR M1 bands normalized for α-tubulin. **(B)** scatter plot with the single values for each patient is shown. **(C)** a representative Western blotting experiments is shown. * p< 0.05 vs Ig ctr.

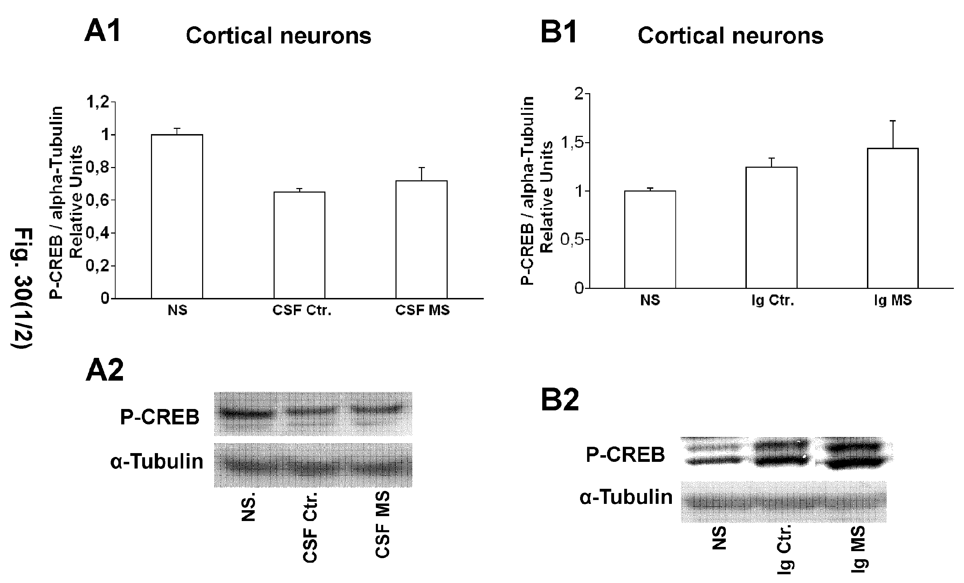

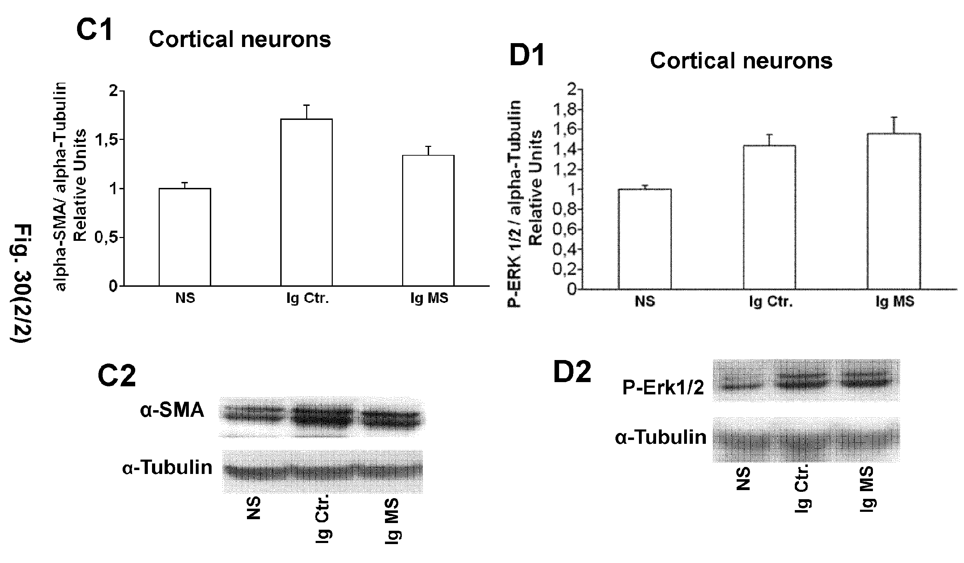

**Fig. 30****: Effects of biological fluids from Control or MS patients on P-CREB, α-SMA and P-ERK1/2 levels in mouse cortical neurons (Example 10)**
   **(A)** Mouse cortical neurons were treated for 30min with 30% CSF from Ctr. or MS patients and then a harvested for Western blotting analysis of P-CREB levels. Mouse cortical neurons were treated for 30min with 200µg/ml of serum IgG from Control or MS patients and then harvested for Western blotting analysis of **(B)** P-CREB, **(C)** α-SMA or **(D)** P-ERK1/2 levels. The histograms (A1, B1, C1, D1) show the mean ± SEM values relative to not stimulated (NS) samples obtained by densitometric analysis of protein bands normalized for α-tubulin. In the lower part of the figures (A2, B2, C2, D2) the representative Western blotting experiments are shown.

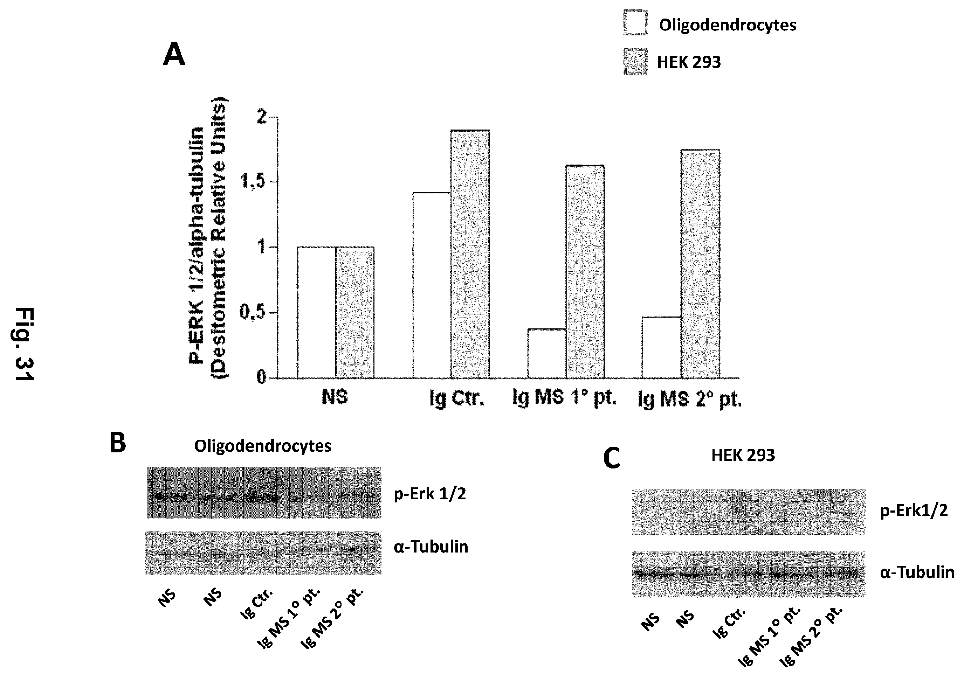

**Fig. 31****: Differential effects of serum IgG from MS patients on P-ERK1/2 levels in M03-13 or HEK293 cells treated with PMA (Example 10)**
   M03-13 or HEK293 cells were treated for 24h with 200µg/ml of serum IgG from Ctr. or MS patients in the presence of 100nM PMA in medium without serum and then P-ERK1/2 levels were analyzed by Western blotting analysis. The histogram **(A)** shows the values relative to NS samples of 1 Control and 2 MS patients obtained by densitometric analysis of protein bands normalized for α-tubulin. In the lower part of the figures **(B, C)** the representative Western blotting experiments are shown.

### Detailed description of the invention

### MATERIALS AND METHODS

### Cell cultures

*M03-13 cells -* The M03-13 cells are an immortal human-human hybrid cell line (CELLutionBiosystem Inc., Canada) with the phenotypic characteristics of primary oligodendrocytes, derived from the fusion of human rhabdomyosarcoma with oligodendrocytes obtained from adult human brain. They were grown in Dulbecco's Modified Eagles Medium (DMEM), containing 4.5g/L glucose, supplemented with 10% FBS, 100 U/ml penicillin and 100 µg/ml streptomycin.

*HEK293 -* The HEK293 derived from human embryonic kidney cells (American Type Culture Collection, ATCC, USA), were grown in Dulbecco's Modified Eagles Medium (DMEM), containing 4.5g/L glucose, supplemented with 10% FBS, 100 U/ml penicillin and 100 µg/ml streptomycin.

*Primary Cultures of mouse cortical neurons -* Cortical neuronal cultures were prepared from foetal mice (14-16 days gestation). Embryos were decapitated, and the neocortices were dissected, dissociated and plated in Eagle's minimal essential medium (Gibco, Auckland, New Zealand) supplemented with 20 mmol glucose (Sigma Chemical), 2 mmol glutamine (Gibco), 5% foetal bovine serum (Gibco) and 5% horse serum (Gibco) on 96-well culture plates, which were prepared by incubation overnight with 40 g/ mL poly-d-lysine (Sigma-Aldrich) and subsequent rinsing with water. Glial cell replication was halted at 5 days in vitro (DIV), by 2 days exposure to 10 µmol cytosine arabinoside. Cultures were kept in a 5% CO2 and 95% air atmosphere at 37 °C; at 7 DIV they were shifted into a growth medium identical to the plating medium, but lacking foetal serum. Cultures were used for experiments at 13 - 14 DIV.

The cells were kept in a 5% CO₂ and 95% air atmosphere at 37°C.

### Oligodendrocyte cell genetically modified to express a detectable marker

Authors generated clones of human oligodendrocytes, expressing the firefly luciferase (de Wet et al., 1997), or the Green Fluorescent Protein (GFP) gene (Chalfie et al., 1994) under the control of anyone of MBP, α-SMA, Olig-2 or mAChR M1 promoters (see below for gene accession numbers).
**Alfa-SMA:** accession number NM_001613; GeneID:59
   <http://www.ncbi.nlm.nih.gov/sites/entrez?db=gene&cmd=Retrieve&dopt=full_rep ort&list_uids=59>;
   1kb 5' flanking region (promoter), region from: 90750147 to: 90751147.
**MBP:** accession number NM_001025081.1; GeneID:4155
   <http://www.ncbi.nlm.nih.gov/sites/entrez?db=gene&cmd=Retrieve&dopt=full_rep ort&list_uids=4155,
   <http://www.ncbi.nlm.nih.gov/sites/entrez?db=gene&amp;cmd=Retrieve&amp;dopt =full_report&amp;list_uids=4155>;
   1kb 5' flanking region (promoter), region from: 74843774 to: 74844774.
**mAchR M1:** accession number NM_000738.2; GeneID:1128
   <http://www.ncbi.nlm.nih.gov/sites/entrez?db=gene&cmd=Retrieve&dopt=full_rep ort&list_uids=1128>;
   1kb 5' flanking region (promoter), region from: 62688012 to a: 62689012.
**MAG:** accession number NM_001199216.1; GeneID:4099
   <http://www.ncbi.nlm.nih.gov/sites/entrez?db=gene&cmd=Retrieve&dopt=full_rep ort&list_uids=4099>;
   1kb 5' flanking region (promoter), region from: 35782989 to: 35783989.
**Olig-1:** accession number NM_138983.2; GeneID:116448
   <http://www.ncbi.nlm.nih.gov/sites/entrez?db=gene&cmd=Retrieve&dopt=full_rep ort&list_uids=116448>;
   1kb 5' flanking region (promoter), region from:34442450 to: 34443450.
**Olig-2:** accession number NM_005806.3; GeneID:10215
   <http://www.ncbi.nlm.nih.gov/sites/entrez?db=gene&cmd=Retrieve&dopt=full_rep ort&list_uids=10215>;
   1kb 5' flanking region (promoter), region from: 34398239 to: 34399239.

The DNA sequences corresponding to 1kb 5' flanking region (promoter) of the MBP, Olig-2, or mAChR M1 genes (see below) were inserted in the firefly luciferase reporter vector pGL3.0 Basic (Promega) or GFP reporter vector. The MO3-13 cells were transfected with recombinant vectors using Lipofectamine 2000 (Invitrogen).

Such cells represent a cell system for easy and fast detection of factors or drugs or molecules modifying oligodendrocyte differentiation measured by luminometry or by flow cytometric analysis for luciferase or GFP, respectively.

### M03-13 cell differentiation

Differentiation of M03-13 cells was obtained using two different protocols:
Protocol 1- Incubating the cells in medium with 0-0.2% Foetal Bovine Serum (FBS, Sigma) in the presence of 100nM PMA (Sigma-Aldrich) (time range: 30min-4 days);
Protocol 2- Incubating the cells with 100-200 µM H₂O₂ in complete medium (time range: 30min-4 days).

### Antibodies

Anti MBP (Cat. # AB980), anti P-CREB (Cat. # 06-519), anti PLP (Cat# AB 15454), anti MAG clone 513 (Cat #MAB1567),anti Olig-1 (Cat. # MAB5540) and anti Olig-2 (Cat. # AB9610) antibodies were purchased by Millipore. Anti α-SMA(Cat. # A5228) and anti mAchR (Cat# M9808) antibodies were purchased by SIGMA. Anti P-ERK1/2 (E-4) antibodies were purchased by Santa Cruz Biotechnology (Cat. # sc-7383).

### Western blotting analysis

Total cells lysates were obtained in RIPA buffer (50 mM Tris-HCl, pH 7.5, NaCl 150 mM, 1% NP40, 0.5% deoxycholate, 0.1% SDS) containing 2.5 mM Na-pyrophosphate, 1 mM β-glycerophosphate, 1 mM NaVO₄, 1 mM NaF, 0.5 mM PMSF, and a cocktail of protease inhibitors (Roche, USA). The cells were kept for 15 min at 4°C and disrupted by repeated aspiration through a 21-gauge needle. Cell lysates were centrifuged for 10 min at 11,600xg and the pellets were discarded. Fifty micrograms of total proteins were subjected to SDS-PAGE under reducing conditions. After electrophoresis, the proteins were transferred onto a nitrocellulose filter membrane (Biorad, UK) with a Trans-Blot Cell (Bio-Rad Laboratories, UK) and transfer buffer containing 25 mM Tris, 192 mM glycine, 20% methanol. Membranes were placed in 5% non-fat milk in phosphate-buffered saline, 0.5% Tween 20 (PBST) at 4°C for 2 hr to block the nonspecific binding sites. Filters were incubated with specific antibodies before being washed three times in PBST and then incubated with a peroxidase-conjugated secondary antibody (GE-Healthcare, UK). After washing with PBST, peroxidase activity was detected with the ECL system (GE-Healthcare, UK).

The filters were also probed with an anti α-tubulin antibody (Sigma, USA). Protein bands were revealed by ECL and, when specified, quantified by densitometry using ScionImage software. Densitometric values were normalized to α-tubulin.

### Flow cytometric analysis of Mielin Basic Protein

Cells were grown to semiconfluency in 60-mm culture dishes. After trypsin detachment, 5 x 10⁵ cells are suspended in 1 mL of phosphate buffered saline (PBS) and fixed overnight with 1% formaldehyde at room temperature. Next, cells were permeabilized with 0.1% Triton X-100 for 40 min at 4°C, washed 4x with PBS containing 2% FBS, 0.01% NaN3, 0.1% Triton X-100 (buffer A), and incubated for 45 min at 4° C with 1 : 50 dilution of rabbit polyclonal anti-human MBP Ig. The cells were then washed twice with the same buffer and incubated for 45 min at 4°C with Cy3-conjugated anti-(rabbit IgG) Ig at 1 : 50 dilution. Control cells were incubated with Cy3-conjugated anti-(rabbit IgG) Ig alone. After two washes in buffer A, cells were resuspended in PBS and analyzed by flow cytometry using FACSCAN (BD, Heidelberg, Germany) and WINMDI software.

### Cell fractionation for cytosolic MBP levels detection

Cells, grown to semiconfluence in 100-mm culture dishes in complete medium, were collected by scraping them into a buffer containing 100 mM KCI, 3 mM NaCl, 3,5 mM MgCl₂, 1.25 mM EGTA, 10 mM Pipes 2 mM NaVO₄, 10 mM phenylarsine oxide, 5 mM NaF, and the cocktail of protease inhibitors. Cells were then disrupted by sonication (2-10 sec pulses at 100W) and centrifuged at 600 x g for 10 min. Next, the supernatants were centrifuged at 100,000 x g for 45 min. Fifty micrograms of cytosol proteins were subjected to Western blotting analysis for myelin basic protein (MBP) as described above.

### Real Time PCR analysis

RNA isolation and real-time PCR were performed as follow: total RNA was extracted using TRI-reagent according to the protocol provided by the manufacturer (Sigma, USA). Total RNA (4 µg) was reverse transcribed with Omniscript Reverse Transcriptase (Quiagen, USA) by oligo-dT primers for 60 min at 37 °C in 40 µl reaction volumes. Real-time PCR was performed with an ABI 5700 or ABI PRISM-7900HT Sequence Detection System (Applied Biosystems Inc., USA). Reactions were carried out in 96-well optical reaction plates in a 50 µl final volume containing 25 µl of the SYBR-Green (Applied Biosystems Inc., USA) PCR master mix, 1,25 µl of each gene-specific primer, 40 ng of sample cDNA. Gene-specific primers were designed to selectively amplify α-SMA and MBP and relative expression values were normalized using glucose-6-phosphate dehydrogenase (G6PD). The SYBRGreen (Applied Biosystems Inc., USA) fluorescence was measured at each extension step. The threshold cycle (Ct) value reflects the cycle number at which the fluorescence measurement reached an arbitrary threshold. Melting curve analysis was performed to determine the specificity of the reaction. Real-time PCR was conducted in triplicate for each sample and the mean value was calculated. Primers for human α-SMA and MBP, and G6PD used are the following:
α-SMA Forward: CTG TTC CAG CCA TCC TTC AT (SEQ ID No. 1)
α-SMA Reverse: TCA TGA TGC TGT TGT AGG TGG T (SEQ ID No. 2)
MBP Forward: GGG TCT TCC TGG AGA TTT GGT (SEQ ID No. 3)
MBP Reverse: GCT GTG GTT TGG AAA CGA GGT T (SEQ ID No. 4)
Human G6PD Forward: ACA GAG TGA GCCC TTC TTC AA (SEQ ID No. 5);
Human G6PD Reverse: ATA GGA GTT GCG GGC AAA G (SEQ ID No. 6)

### Immunofluorescence confocal microscopy

M03-13 cells were grown on glass coverslip under culture conditions described in the specific experiments. Then, the medium was removed and cells immediately fixed in 3.7% paraformaldehyde in PBS with 2% sucrose for 5 min at 22°C.

For olig-2 staining, after 2 washes in PBS with 2% sucrose, cells were permeabilized for 5 min at 4°C with 0.1% Triton X-100 in 20 mM Hepes, 300 mM sucrose, 50 mM NaCl, 3 mM MgCl₂. The cells, after blocking with 20% goat serum in PBS for 45min at 4°C, were labelled with primary rabbit polyclonal anti human olig-2 antibody. The cells were washed and labelled with secondary Cy3-conjugated anti rabbit IgG (Jackson ImmunoResearch, USA). For MBP and α-SMA staining, after fixation, the cells were permeabilized for 10 min at 4°C with 0.01% Saponin (Sigma-Aldrich, from quillaja bark) in PBS. The cells, after blocking with 20% FBS in PBS with 0.01% Saponin for 30 minutes at 4°C, were labelled with primary rabbit-polyclonal anti human MBP antibody or with primary mouse-polyclonal anti human α-SMA antibody. The cells were washed and labelled with secondary Cy3-conjugated anti-rabbit IgG (Jackson ImmunoResearch, USA) or with secondary Cy2-conjugated anti-mouse IgG (Jackson ImmunoResearch, USA).

For all staining, controls were incubated with secondary antibodies alone. After treatment with nuclear marker, 4',6-diamidino-2-phenylindole (DAPI), the coverslips were briefly washed first, in PBS and then in distilled water, and finally mounted on glass slides for microscopy examination. Cells were analyzed with a Zeiss LSM 510 Meta laser scanning confocal microscope. Images were analysed using the ImageJ software: the threshold was set on the maximum fluorescence value of the sample treated only with secondary antibody and, for each sample, were quantify 25 cells.

### Purification of Immunoglobulins

The purification of IgG fractions from serum of MS and control subjects was carried out by affinity chromatography on A/G Sepharose columns (Pierce, Rockford, IL). The protein concentration of immunoglobulin fractions thus prepared was assessed spectrophotometrically and used in oligodendrocyte differentiation cell models.

### Patients

In the study were included men and women between 15 and 50 years of age who meet all the following criteria:
- diagnosis of relapsing/remitting MS, according to McDonald criteria;
- an Expanded Disability Scale Score (EDSS) between 0 and 5.0;
- lesions detected by MRI compatible with the diagnosis of multiple sclerosis;
- at least one acute episode in the last 12 months.

Control samples include other neurological disorders affected patients (including inflammatory, degenerative diseases not involving direct or indirect de-myelinization, i. e.: cerebral cancers, stroke, vasculitis, etc) that need differential diagnosis with multiple sclerosis. Control patients were selected by sex and age to be similar to multiple sclerosis patients.

All the patients were subjected to CSF collection by lumbar puncture to execute the routine laboratory analysis in the hospital where they were hospitalized. A quantity of 1-2 ml of CSF was sent to our laboratories to carry out the investigations of our interest. At the same time a blood sample, from each patients, was collected to purify the IgG fractions from blood serum. Patients gave written informed consent before any study-related procedures was performed.

### Statistical analysis

Statistical differences were evaluated using a Student's *t*-test for unpaired samples.

### RESULTS

The authors have developed 2 different protocols to differentiate human oligodendrocyte M03-13 cells. Cells were grown i) in medium with 0-0.2% serum in presence of 100nM PMA or ii) in complete medium in presence of low doses (200µM) of hydrogen peroxide. Then, the expression or the localization of specific differentiation markers was evaluated at different times.

The authors first evaluated the effects of 30 min exposure to 100 nM PMA on P-CREB and P-ERK1/2 levels in cells pre-incubated for 16h in medium containing 0.2% FBS. It is known that both proteins are signalling molecules involved in oligodendrocyte differentiation (Afshari et al., 2001; Chandran et al., 2003). Fig. 1A shows that PMA significantly increases the levels of P-CREB and P-ERK1/2.

Different experimental evidences suggest that α-Smooth Muscle Actin (α-SMA) can be considered a negative oligodendrocyte differentiation marker; in Schwann cell-like cells derived from the bone marrow, the withdrawal of differentiation stimulus is accompanied with the generation of SMA expressing cells (Shea et al., 2010); moreover, the treatment of neural stem cells derived from rat spinal cord with Bone Morphogenetic Protein (BMP) inhibits oligodendrocyte formation and generate SMA expressing cells (Chandran, 2003). Therefore, the authors evaluated the expression of P-CREB, P-ERK1/2, α-SMA and Olig-2 protein levels after 24h of differentiation with 100 nM PMA in absence of serum. As can be shown in Fig. 1B, P-ERK1/2, P-CREB and Olig-2 levels increased thus, were induced by differentiation. On the contrary, differentiation of the cells was accompanied with a decrease inα-SMA levels. The levels of MBP were then evaluated by flow cytometric analysis at 24h and 4 days of differentiation. Fig. 1C shows that PMA 100nM in medium without serum induces a time-dependent increase in MBP protein levels.

Moreover, the authors evaluated the effects of PMA on the translocation of the specific oligodendrocyte differentiation marker olig-2 from nucleus to cytosol. This protein is located in the nucleus during the early stage of oligodendocyte maturation steps and then migrates from the nucleus to the cytoplasm, becoming inactive. Fig. 1 D shows confocal microscopy images of M03-13 cells immunostained with antibodies directed against olig-2, in not differentiated cells the signal is mainly localized in the nucleus while after 24h of treatment with 100 nM PMA in medium without serum a cytoplasmic staining of the cells became evident. The levels of mAchR M1 protein were measured at 24h and 4 days of differentiation with 100 nM PMA in absence of serum. As shown in Fig. 1E, mAchR M1 level increased after 4 days of differentiation.

The authors used the second protocol to differentiate the oligodendrocytes, stimulating M03-13 cells with low doses of hydrogen peroxide for 30 min-4 days, then they evaluated the expression of oligodendrocyte differentiaton markers.

The authors found that stimulation of cells with 200 µM H₂O₂ for 4 days in complete medium decreasesα-SMA and induces P-CREB levels; the P-ERK1/2 levels were, instead, not affected. The generic antioxidant N-Acetyl-Cysteine (NAC) reverted hydrogen peroxide effects, while the inhibitor of the membrane NADPH oxidase AEBSF, did not (Fig. 2A). Hydrogen peroxide stimulation also increases total MBP protein levels as measured by flow cytometric analysis (Fig. 2B). Following differentiation, MBP translocates from cytosol to lipid rafts membrane microdomains; therefore the authors, as a further marker of cell differentiation, also determined cytosolic MBP levels by cell fractionation and Western blotting analysis. Treatment of the cells for 4 days with 200 µM hydrogen peroxide in complete medium or 100 nM PMA in absence of serum decreases cytosolic MBP levels according with its translocation to the membrane compartment (Fig. 2C). Fig. 2 D shows confocal microscopy images of M03-13 cells immunostained with antibodies directed against olig-2; in not differentiated cells the signal is mainly localized in the nucleus while after the treatment with hydrogen peroxide a cytoplasmic staining of the cells became evident already after 30 min increasing further at 24h.

The effects of 100 nM PMA in serum-free medium or of 200 µM hydrogen peroxide in complete medium for 1 and 4 days on *α-SMA, MBP* or Olig-2 protein levels were also evaluated by confocal microscopy analysis.

Figure 2E shows that the signal derived from antibodies directed against *α-SMA* decreased both in cells stimulated by PMA and with H₂O₂, compared to not differentiated (N.D.) cells. In addition, in M03-13 cells immunostained with antibodies directed against *MBP,* the signal increased in cells treated with 100 nM PMA in serum-free medium, compared with not differentiated cells and accumulated in cellular processes either at 1 day or 4 days of treatment; similar increase and cellular pattern of fluorescent signal were observed in the cells treated with 200 µM H₂O₂ in complete medium (Fig. 2F). Finally, in cells treated with 100 nM PMA in serum-free medium, as well as in cells treated with 200 µM H₂O₂ in complete medium, *Olig-2* fluorescent signal progressively increased at 1 day and 4 days compared to not differentiated cells (Fig. 2G).

Overall these data demonstrate that stimulation of cells with low doses of hydrogen peroxide induces the differentiation of oligodendrocyte precursors to differentiated cells.

In conclusion, the authors have studied the oligodendrocyte differentiation markers P-CREB, P-ERK1/2, α-SMA, MBP, mAchR M1 and olig-2 and have observed that:
- P-CREB: increases with differentiation
- P-ERK1/2: increases with differentiation
- α-SMA: decreases with differentiation
- MBP: increases with differentiation
- mAchRM1: increases with differentiation
- Olig-2: translocates from nucleus to cytosol and increases with differentiation Therefore, such markers are useful for diagnosis, prognosis, to follow up efficacy of a therapy and for the development of treatment for multiple sclerosis.

The following examples demonstrate that incubation of human oligodendrocyte cells with biological fluids from MS patients (CSF or Serum IgG) inhibits cell differentiation.

### Example 1: CSF from MS patients inhibits the differentiation of M03-13 cells after 24h of treatment and after 4 days of treatment

### 24h of treatment

Oligodendrocytes were treated for 24h with 100 nM PMA in medium without serum in absence (Differentiated, Diff.) and presence of 30% CSF from Control (Ctr) or MS patients and then subjected to Western blotting analysis of P-ERK1/2 (Fig. 3), α-SMA (Fig. 4) and Olig-2 (Fig. 5) levels. Flow cytometric analysis of MBP protein levels (Fig. 6), PCR analysis of MBP mRNA levels (Fig. 7), immunofluorescence staining and confocal microscopy analysis of Olig-2 (Fig. 8 and 10) and MBP (Fig. 9) were also performed.

In differentiated oligodendrocytes in presence of CSF from MS patients, the levels of P-ERK1/2 and Olig-2 were significantly lower and the levels of α-SMA significantly higher compared to Controls (Fig. 3,4, 5, respectively). The protein and mRNA levels of MBP were significantly lower compared to those in presence of CSF from control patients (Fig. 6, 7, 9). Finally, CSF from MS patients completely inhibits Olig-2 translocation from nucleus to cytosol in oligodendrocytes differentiated for 24h (Fig. 8). Moreover confocal analysis confirmed that Olig-2 levels were significantly lower compared to those in presence of CSF from control patients (Fig. 10).

### 4 days of treatment

Oligodendrocytes were treated for 4 days with 100nM PMA in medium without serum in absence (Differentiated, Diff.) and presence of 30% CSF from Control (N) or MS patients and then subjected to confocal analysis of α-SMA, MBP, and Olig-2. In oligodendrocytes differentiated in presence of CSF from MS patients the levels of α-SMA (Fig. 11) were significantly lower and the levels of MBP (Fig. 12) and Olig-2 (Fig. 13) significantly higher compared to those in the CSF of control patients.

### Example 2: 30 min exposure to CSF from MS patients inhibits late differentiation of M03-13 cells

It is known that CREB is a mediator of signal transduction pathways that operates at different stages of oligodendrocyte development and differentiation. In particular, the expression pattern of CREB protein suggests a role of this protein at the later stages of differentiation. For this reason, the authors have also evaluated the effects of CSF from MS patients on P-CREB protein levels in oligodendrocytes differentiated for 4 days. Thirty min exposure of the cells with CSF from MS patients reduces P-CREB protein levels compared to CSF from Controls (Fig. 14).

### Example 3: Serum IgGs from MS patients inhibit differentiation: decrease of P-ERK1/2 levels

The authors have analyzed the effects of serum IgG from MS patients on P-ERK1/2 levels. Serum IgG from MS patients reduced P-ERK1/2 levels compared to those found in samples incubated in the presence of IgG of control patients either at 30 min (Fig. 15) or 24h of differentiation (Fig. 16).

### Example 4: Serum IgGs from MS patients inhibit differentiation: decrease of P-CREB levels

The authors have analyzed the effects of serum IgG from MS patients on P-CREB levels after 30 min of differentiation. Serum IgG from MS patients reduced P-CREB levels compared to those in Controls (Fig. 17).

### Example 5: Serum IgGs from MS patients inhibit differentiation: decrease of Olig-2 levels

The authors have analyzed the effects of serum IgG from MS patients on Olig-2 levels. Serum IgG from MS patients reduced Olig-2 levels compared to those found in samples incubated in the presence of serum IgG of control patients at 24h of differentiation (Fig. 18).

### Example 6: Serum IgGs from MS patients inhibit differentiation: increase of α-SMA protein and mRNA levels

The authors have analyzed the effects of serum IgG from MS patients on α-SMA protein and mRNA levels after 24h of differentiation.

Serum IgG from MS patients increased α-SMA protein levels after 1d (Fig. 19) **and 4d** (Fig. 21) differentiation and mRNA (Fig. 20) levels after 1d differentiation compared to those of samples incubated with serum IgG of control patients.

### Example 7: Serum IgGs from MS patients inhibit differentiation: decrease of MBP protein levels

The authors have analyzed the effects of serum IgG from MS patients on total (by flow cytometry and confocal analysis) and cytosolic MBP protein levels by Western blotting analysis, after 24h of differentiation.

Either total or cytosolic MBP protein levels were significantly lower in cells treated with serum IgG from MS compared to those in samples incubated with serum IgG of control patients (Fig. 22, 23, 24).

In addition, the effects of serum IgG from MS patients on total MBP protein levels by confocal analysis, after 4 days of differentiation were measured. MBP protein levels were significantly lower in cells treated with serum IgG from MS compared to those in cells treated with IgG of control patients (Fig. 25).

### Example 8: Serum IgGs from MS patients interfere with translocation from nucleus to cytosol of Olig-2 after 24 of differentiation and decrease Olig-2 levels after 24h and 4 days of differentiation

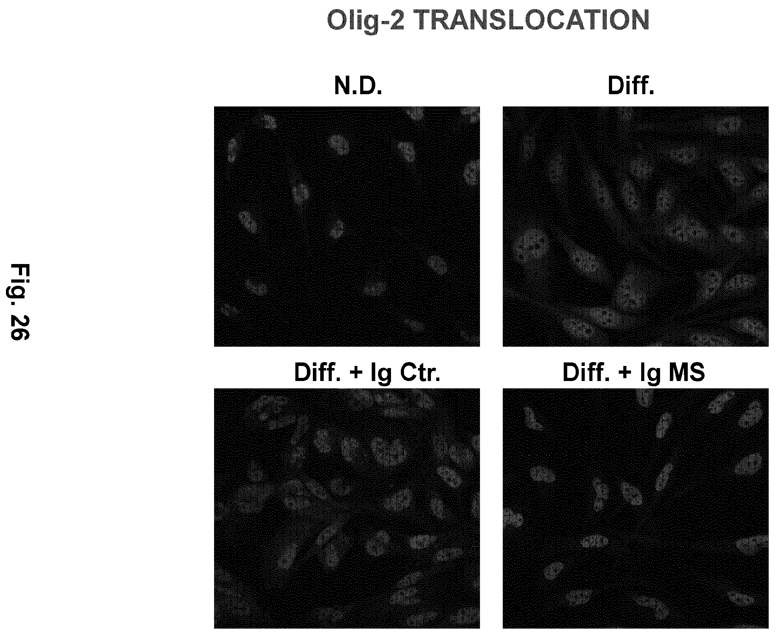

The authors have analyzed the effects of serum IgG from MS patients on Olig-2 localization by confocal microscopy. Serum IgG from MS patients inhibits the translocation of Olig-2 from nucleus to cytosol after 24h of differentiation (Fig. 26).

In addition, the effects of serum IgG from MS patients on Olig-2 protein levels by confocal analysis, after 1 and 4 days of differentiation were measured.

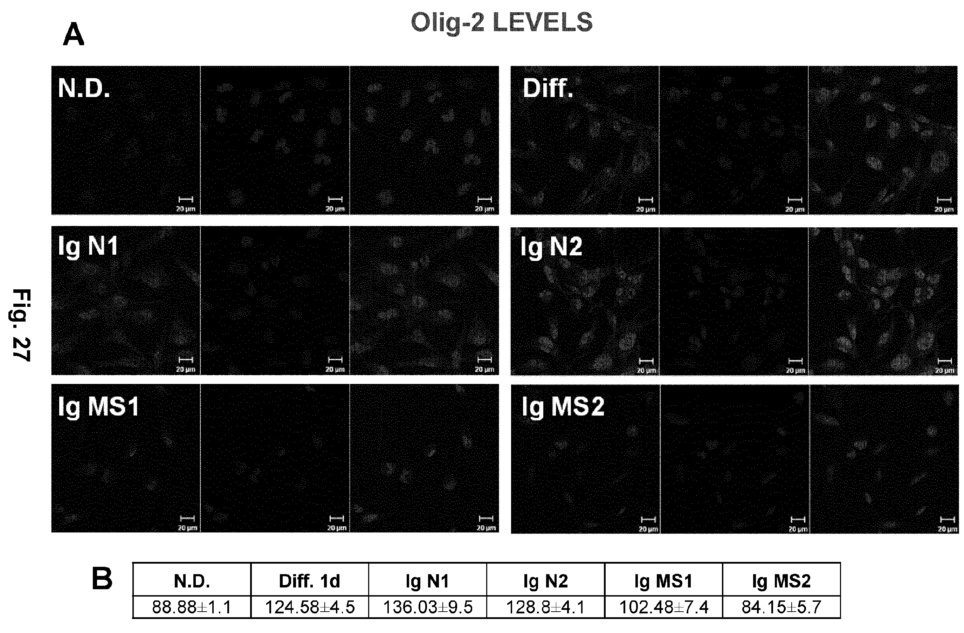

Olig-2 protein levels were significantly lower in cells treated with serum IgG from MS compared to those in cells treated with serum IgG from control patients after 1d (Fig. 27) **and 4d** (Fig. 28).

### Example 9: Serum IgGs from MS patients inhibit differentiation: decrease in mAchR M1 protein levels

The authors have analyzed the effects of serum IgG from MS patients on mAchR M1 protein levels after 4 days of differentiation.

Serum IgG from MS patients decreased mAchR M1 protein levels compared to those found in samples from serum IgGs of control patients (Fig. 29).

### Example 10: Cell specificity

To ascertain whether the effects of biological fluids from MS patients on several differentiation markers were specific for oligodendrocyte cells, the authors carried out experiments on mouse cortical primary neurons and on HEK293 cells.

The mouse neurons were incubated for 30 min with CSF from MS or Control patients and P-CREB levels were analyzed (Fig. 30A). In another set of experiments mouse neurons were stimulated for 30 min with serum IgGs from MS and Control patients and P-CREB (Fig. 30B), α-SMA (Fig. 30C) or P-ERK1/2 (Fig. 30D) levels were analyzed. None of these experiments highlighted differences in the expression of differentiation markers between the two experimental groups.

The authors also tested the effects of serum IgGs from one Control and from two MS patients on P-ERK1/2 levels after 24 h of differentiation in both M03-13 cells and HEK293 cells. Fig. 31 shows that serum IgGs from MS patients decreased P-ERK1/2 protein levels in oligodendrocyte cells compared to differentiated cells, while did not affect P-ERK1/2 levels in HEK293 cells demonstrating the cell specificity of the effects of serum IgG from MS patients.

In conclusion, the authors have studied the oligodendrocyte differentiation markers P-CREB, P-ERK1/2, α-SMA, MBP, mAchR M1 and olig-2 in the presence of differentiation stimulus and MS biological samples and have observed that:
- P-CREB: decrease in the presence of MS biological samples
- P-ERK1/2: decrease in the presence of MS biological samples
- α-SMA: increases in the presence of MS biological samples
- MBP: decreases in the presence of MS biological samples
- mAchR M1 increases with differentiation
- Olig-2: translocates from nucleus to cytosol and increases with differentiation

### Bibliographic references

- Achten E, DeblaereK.(2008) Eur J Radiol,65: 211-3.
- Afshari FS, Chu AK, Sato Bigbee CS (2001) JNeurosc Res,66: 37-45.
- Chalfie M, Tu Y, Euskirchen G, Ward WW, PrasherDC(1994) Science, 263: 802-5.
- Chandran S, et al.(2003) Development,130:6599-609.
- Chang A, Tourtellotte WW, Rudick R, Trapp BD(2002) N Engl J Med,346:165-73.
- de Wet J R, Wood KV, DeLuca M, Helinski DR, and Subramani S. (1987) Mol Cell Biol, 7: 725-37.
- Fragoso G, et al.(2004) J Neurochem,90:392-404.
- Luque FA, Jaffe SL. (2007) Int Rev Neurobiol, 79:341-56.
- Marrie RA. (2004) Lancet Neurol, 3: 709-718.
- Murdoch D, Lyseng-Williamson KA. (2005) Drugs, 65:1295-312.
- Noseworthy JH, et al.(2000) NEngl J Med,343:938-52.
- Shea GKH, Tsui AYP, Chan YS, Shum D KY. (2010) ExpNeurol,224: 448-458.
- Trapp BD, Peterson J, Ransohoff RM, et al. (1998) N Engl J Med, 338:278-285.
- Trojano M, Paolicelli D. (2001) NeurolSCi,22Suppl 2:S98-102.
- Wolswijk G. (1998) J Neurosc,18:601-9.
- Young IR, Hall AS, Pallis CA, Legg NJ, Bydder GM, Steiner RE. (1981) Lancet,2: 1063-6.

## Claims

1. A method for diagnosis and/or prognosis of multiple sclerosis in a subject,comprising the steps of:
a) incubating oligodendrocyte cells in the presence of a differentiation stimulus with a suitable amount of a biological sample obtained from the subject;
b) measuring the amount of one or more molecular species in said incubated oligodendrocyte cells, one of said molecular species being mAChR M1;
c) comparing the measured amount of said molecular species to a proper control and optionally
d) assessing the cellular localization of olig-2 and comparing the cellular localization of olig-2 to a proper control.

2. The method according to claim 1 further comprising measuring the amount of one or more molecular species belonging to the group of: olig-2, P-CREB, MBP, or an introduced detectable marker gene under the control of one promoter selected from the group of mAChR M1, olig-2, P-CREB, MBP genes and comparing the measured amount of said molecular species to a proper control.

3. A method for monitoring the efficacy of a therapeutic agent and/or screening for a candidate therapeutic agent for multiple sclerosis, comprising the steps of:
a) incubating oligodendrocyte cells in the presence of a differentiation stimulus with a suitable amount of a biological sample obtained from the subject, and with a therapeutic agent or a candidate therapeutic agent, respectively, for multiple sclerosis;
b) measuring the amount of one or more molecular species in said incubated oligodendrocyte cells, one of said molecular species being mAChR M1;
c) comparing the measured amount of said molecular species to a proper control and optionally
d) assessing the cellular localization of olig-2 and comparing the cellular localization of olig-2 to a proper control.

4. The method according to claim 3 further comprising measuring the amount of one or more molecular species belonging to the group of: olig-2, P-CREB, MBP or an introduced detectable marker gene under the control of one promoter selected from the group of mAChR M1, olig-2, P-CREB, MBP genes and comparing the measured amount of said molecular species to a proper control.

5. The method according to any of previous claims wherein the amount of at least two molecular species is measured.

6. The method according to any of previous claims wherein the amount of at least three molecular species is measured.

7. The method according to any of previous claims wherein the amount of all of molecular species is measured.

8. The method according to any of previous claims wherein the detectable marker is a luciferase or GFP.

9. The method according to any of previous claims further comprising measuring the amount of at least another molecular species belonging to the group of: P-ERK1/2 or α-SMA.

10. The method according to any of previous claims wherein the biological sample is cerebrospinal fluid, blood sample or serum sample or Ig-comprising derivatives thereof.

11. The method according to any one of previous claims wherein the differentiation stimulus consists of incubating oligodendrocyte cells in the presence of: a) Phorbol Myristate Acetate (PMA); b) hydrogen peroxide; c) low serum medium; d) cyclic adenosine monophosphate (cAMP) analogs; e) adenylate cyclase activators; f) thyroid hormones as 3,5,3'-L-triiodothyronine (T3) and thyroxin (T4); g) ERB B inhibitors; h) nuclear receptor ligand; or i) nucleoside analogs.

12. Use of a kit for the diagnosis and/or prognosis of multiple sclerosis or to monitor the efficacy of a therapy for multiple sclerosis according to the method of claims 1-11 comprising means to measure mAChR M1 and P-CREB.

13. The use according to claim 12 wherein said kit further comprises means to measure olig-2 and/or MBP and/or P-ERK1/2 and/or α-SMA.

## Patentansprüche

1. Verfahren für die Diagnose und/oder Prognose von multipler Sklerose in einem Individuum, das die folgenden Schritte umfasst:
a) Inkubieren von Oligodendrozytenzellen im Beisein eines Differenzierungsreizes mit einer geeigneten Menge einer von dem Individuum erhaltenen biologischen Probe;
b) Messen der Menge mindestens einer Molekülart in den inkubierten Oligodendrozytenzellen, wobei es sich bei einer der Molekülarten um mAChR M1 handelt;
c) Vergleichen der gemessenen Menge der Molekülart mit einer geeigneten Kontrolle und wahlweise
d) Beurteilen der zellulären Lokalisierung von Olig-2 und Vergleichen der zellulären Lokalisierung von Olig-2 mit einer geeigneten Kontrolle.

2. Verfahren nach Anspruch 1, welches ferner das Messen der Menge mindestens einer Molekülart umfasst, die zu der Gruppe von Olig-2, P-CREB, MBP oder einem eingeführten detektierbaren Markergen unter der Kontrolle eines Promotors, der aus der Gruppe mAChR M1, Olig-2, P-CREB, MBP-Gene ausgewählt ist, gehört, und Vergleichen der gemessenen Menge der Molekülart mit einer geeigneten Kontrolle.

3. Verfahren zum Überwachen der Wirksamkeit eines therapeutischen Agens und/oder zum Screening auf ein therapeutisches Kandidatenagens gegen multiple Sklerose, das die folgenden Schritte umfasst:
a) Inkubieren von Oligodendrozytenzellen im Beisein eines Differenzierungsreizes mit einer geeigneten Menge einer von dem Individuum erhaltenen biologischen Probe und mit einem therapeutischen Agens oder einem therapeutischen Kandidatenagens gegen multiple Sklerose;
b) Messen der Menge mindestens einer Molekülart in den inkubierten Oligodendrozytenzellen, wobei es sich bei einer der Molekülarten um mAChR M1 handelt;
c) Vergleichen der gemessenen Menge der Molekülart mit einer geeigneten Kontrolle und wahlweise
d) Beurteilen der zellulären Lokalisierung von Olig-2 und Vergleichen der zellulären Lokalisierung von Olig-2 mit einer geeigneten Kontrolle.

4. Verfahren nach Anspruch 3, welches ferner das Messen der Menge mindestens einer Molekülart umfasst, die zu der Gruppe von Olig-2, P-CREB, MBP oder einem eingeführten detektierbaren Markergen unter der Kontrolle eines Promotors, der aus der Gruppe mAChR M1, Olig-2, P-CREB, MBP-Gene ausgewählt ist, gehört, und Vergleichen der gemessenen Menge der Molekülart mit einer geeigneten Kontrolle.

5. Verfahren nach einem der vorhergehenden Ansprüche, wobei die Menge von mindestens zwei Molekülarten gemessen wird.

6. Verfahren nach einem der vorhergehenden Ansprüche, wobei die Menge von mindestens drei Molekülarten gemessen wird.

7. Verfahren nach einem der vorhergehenden Ansprüche, wobei die Menge aller Molekülarten gemessen wird.

8. Verfahren nach einem der vorhergehenden Ansprüche, wobei es sich bei dem detektierbaren Marker um eine Luciferase oder GFP handelt.

9. Verfahren nach einem der vorhergehenden Ansprüche, das ferner das Messen der Menge mindestens einer anderen Molekülart umfasst, die zu der Gruppe P-ERK1/2 oder α-SMA gehört.

10. Verfahren nach einem der vorhergehenden Ansprüche, wobei es sich bei der biologischen Probe um Gehirnrückenmarkflüssigkeit, eine Blutprobe oder eine Serumprobe oder um Ig umfassende Derivate davon handelt.

11. Verfahren nach einem der vorhergehenden Ansprüche, wobei der Differenzierungsreiz aus dem Inkubieren von Oligodendrozytenzellen im Beisein von: a) Phorbolmyristatacetat (PMA); b) Wasserstoffperoxid; c) einem Medium mit niedrigem Serumgehalt; d) Analogen von cyclischem Adenosinmonophosphat (cAMP); e) Adenylatcyclase-Aktivatoren; f) Schilddrüsenhormone, z. B. 3,5,3'-L- Triiodthyronin (T3) und Thyroxin (T4); g) ERB B-Inhibitoren; h) einem Kernrezeptorliganden; oder i) Nukleosidanaloge besteht.

12. Verwendung eines Kits für die Diagnose und/oder Prognose multipler Sklerose oder zur Überwachung der Wirksamkeit einer Therapie gegen multiple Sklerose nach dem Verfahren der Ansprüche 1 bis 11, umfassend Mittel zum Messen von mAChR M1 und P-CREB.

13. Verwendung nach Anspruch 12, wobei das Kit ferner Mittel zum Messen von Olig-2 und/oder MBP und/oder P-ERK1/2 und/oder α-SMA umfasst.

## Revendications

1. Procédé de diagnostic et/ou de pronostic de la sclérose en plaques chez un sujet, comprenant les étapes de :
a) incuber des oligodendrocytes en présence d'un stimulus de différenciation avec une quantité appropriée d'un échantillon biologique prélevé du sujet ;
b) mesurer la quantité d'une ou de plusieurs espèces moléculaires dans lesdits oligodendrocytes incubés, l'une desdites espèces moléculaires étant mAChR M1 ;
c) comparer la quantité mesurée de ladite espèce moléculaire à un témoin approprié et facultativement
d) évaluer la localisation cellulaire d'olig-2 et comparer la localisation cellulaire d'olig-2 à un témoin approprié.

2. Procédé selon la revendication 1, comprenant en outre la mesure de la quantité d'une ou de plusieurs espèces moléculaires appartenant au groupe de : olig-2, P-CREB, MBP ou un gène marqueur détectable introduit sous le contrôle d'un promoteur sélectionné dans le groupe constitué des gènes mAChR M1, olig-2, P-CREB, MBP et la comparaison de la quantité mesurée de ladite espèce moléculaire à un témoin approprié.

3. Procédé de surveillance de l'efficacité d'un agent thérapeutique et/ou de criblage d'un agent thérapeutique candidat pour la sclérose en plaques, comprenant les étapes consistant à :
a) incuber des oligodendrocytes en présence d'un stimulus de différenciation avec une quantité appropriée d'un échantillon biologique prélevé du sujet, et avec un agent thérapeutique ou un agent thérapeutique candidat, respectivement, pour la sclérose en plaques ;
b) mesurer la quantité d'une ou de plusieurs espèces moléculaires dans lesdits oligodendrocytes incubés, l'une desdites espèces moléculaires étant mAChR M1 ;
c) comparer la quantité mesurée de ladite espèce moléculaire à un témoin approprié et facultativement
d) évaluer la localisation cellulaire d'olig-2 et comparer la localisation cellulaire d'olig-2 à un témoin approprié.

4. Procédé selon la revendication 3, comprenant en outre la mesure de la quantité d'une ou de plusieurs espèces moléculaires appartenant au groupe : olig-2, P-CREB, MBP ou un gène marqueur détectable introduit sous le contrôle d'un promoteur sélectionné dans le groupe constitué des gènes mAChR M1, olig-2, P-CREB, MBP et la comparaison de la quantité mesurée de ladite espèce moléculaire à un témoin approprié.

5. Procédé selon l'une quelconque des revendications précédentes, dans lequel la quantité d'au moins deux espèces moléculaires est mesurée.

6. Procédé selon l'une quelconque des revendications précédentes, dans lequel la quantité d'au moins trois espèces moléculaires est mesurée.

7. Procédé selon l'une quelconque des revendications précédentes, dans lequel la quantité de la totalité des espèces moléculaires est mesurée.

8. Procédé selon l'une quelconque des revendications précédentes, dans lequel le marqueur détectable est une luciférase ou GFP.

9. Procédé selon l'une quelconque des revendications précédentes comprenant en outre la mesure d'au moins une autre espèce moléculaire appartenant au groupe : P-ERK1/2 ou α-SMA.

10. Procédé selon l'une quelconque des revendications précédentes, dans lequel l'échantillon biologique est un échantillon de liquide céphalo-rachidien, un échantillon de sang ou un échantillon de sérum ou des dérivés de ceux-ci comprenant des Ig.

11. Procédé selon l'une quelconque des revendications précédentes, dans lequel le stimulus de différenciation consiste incuber les oligodendrocytes en présence : a) d'acétate et myristate de phorbol (PMA) ; b) de peroxyde d'hydrogène ; c) d'un milieu à faible teneur en sérum ; d) d'analogues de l'adénosine monophosphate cyclique (AMPc) ; e) d'activateurs de l'adénylate cyclase ; f) d'hormones thyroïdiennes telles que la 3,5,3'-L-triiodothyronine (T3) et la thyroxine (T4) ; g) d'inhibiteurs d'ERB B ; h) d'un ligand du récepteur nucléaire ; ou i) d'analogues nucléosidiques.

12. Utilisation d'un kit pour le diagnostic et/ou le pronostic de la sclérose en plaques ou pour surveiller l'efficacité d'une thérapie contre la sclérose en plaques selon le procédé des revendications 1 à 11 comprenant un moyen permettant de mesurer mAChR M1 et P-CREB.

13. Utilisation selon la revendication 12, dans laquelle ledit kit comprend en outre un moyen permettant de mesurer olig-2 et/ou MBP et/ou P-ERK1/2 et/ou α-SMA.


### SEQUENCE LISTING

<110> Avvedimento, Vittorio Enrico Paternò, Roberto Santillo, Mariarosaria
<120> A METHOD FOR DIAGNOSIS, MONITORING THE EFFICACY OF A THERAPY AND FOR DEVELOPMENT OF TREATMENT FOR MULTIPLE SCLEROSIS
<130> PCT115117
<150> RM2010A000654
   <151> 2010-12-13
<160> 6
<170> PatentIn version 3.5
<210> 1
   <211> 20
   <212> DNA
   <213> Artificial Sequence
<220>
   <223> synthetic primer
<400> 1
   ctgttccagc catccttcat 20
<210> 2
   <211> 22
   <212> DNA
   <213> Artificial Sequence
<220>
   <223> synthetic primer
<400> 2
   tcatgatgct gttgtaggtg gt 22
<210> 3
   <211> 21
   <212> DNA
   <213> Artificial Sequence
<220>
   <223> synthetic primer
<400> 3
   gggtcttcct ggagatttgg t 21
<210> 4
   <211> 22
   <212> DNA
   <213> Artificial Sequence
<220>
   <223> synthetic primer
<400> 4
   gctgtggttt ggaaacgagg tt 22
<210> 5
   <211> 21
   <212> DNA
   <213> Artificial Sequence
<220>
   <223> synthetic primer
<400> 5
   acagagtgag cccttcttca a 21
<210> 6
   <211> 19
   <212> DNA
   <213> Artificial Sequence
<220>
   <223> synthetic primer
<400> 6
   ataggagttg cgggcaaag 19